# EUROPEAN PATENT APPLICATION

(11) **EP 4 825 322 A1**
(43) Date of publication of application: **21.10.2026**
(21) Application number: 26172882.8
(22) Date of filing: 16.04.2026
(51) Int. Cl.: A61B 6/03, A61B 6/46, A61B 8/00, A61B 8/08

(54) **IMAGE PROCESSING APPARATUS, IMAGE PROCESSING SYSTEM AND IMAGE PROCESSING METHOD**

(30) Priority: 18.04.2025 JP 2025068498
(71) Applicant: Canon Kabushiki Kaisha, Tokyo 146-8501 (JP)
(72) Inventor: AOYAMA, Gakuto, Tokyo, 146-8501 (JP)
(74) Representative: Marks & Clerk LLP

(57) **Abstract**

An image processing apparatus (100) includes a first acquisition unit (131) that obtains first image data ; a first feature identification unit (132) that identifies a first feature related to a first organ captured in the first image data; a second feature identification unit (134) that identifies, corresponding to imaging range for a second organ that is captured in second image data, a second feature related to imaging range for the second organ in the first image data; a third feature identification unit (135) that identifies a settable range for an imaging parameter in the second imaging method; and an estimation unit (136) that estimates based on the first feature related to the first organ, the second feature related to imaging range for the second organ, and the settable range for an imaging parameter in the second imaging method, an imaging parameter for the second organ in the second imaging method.

## Description

### FIELD

Embodiments described herein relate generally to an image processing apparatus and an image processing method.

### BACKGROUND

At present, as a method for taking medical images of an organ in a human body, an imaging method is available in which the images of one organ are taken via another organ. For example, as a technology for ultrasound imaging of the heart, transesophageal echocardiography (TEE) can be cited. In transesophageal echocardiography, an operator inserts an ultrasound probe from the mouth of the subject into the esophagus and obtains ultrasound images of the heart via the esophagus.

In that case, the ultrasound probe is positioned adjacent to the heart and the aorta, which is close to the heart, via the esophagus; and is positioned behind the heart. Hence, it becomes possible to avoid the influence of the chest wall and the air in the lungs, thereby enabling obtaining clear ultrasound images of the heart. In this way, transesophageal echocardiography plays a significant role in catheter-based therapy meant for widening the arteries clogged due to arteriosclerosis, or in interventional treatment for structural heart disease.

As compared to the commonly-used transthoracic echocardiography (TTE) in which the operator applies an ultrasound probe to the body surface close to the heart and obtains ultrasound images, transesophageal echocardiography is an invasive imaging technique in which a transducer is inserted into the esophagus for obtaining ultrasound images. In transesophageal echocardiography, the operator obtains images by operating the transducer placed inside the esophagus of the subject.

### SUMMARY OF THE INVENTION

According to a first aspect, an image processing apparatus is provided, which includes a first acquisition unit configured to obtain first image data by performing imaging according to a first imaging method; a first feature identification unit configured to identify a first feature related to a first organ captured in the first image data; a second feature identification unit configured to identify, corresponding to imaging range for a second organ that is different than the first organ and that is captured in second image data obtained according to a second imaging method different than the first imaging method, a second feature related to imaging range for the second organ in the first image data; a third feature identification unit configured to identify a settable range for an imaging parameter in the second imaging method; and an estimation unit configured to estimate based on the first feature related to the first organ in the first image data, the second feature related to imaging range for the second organ, and the settable range for an imaging parameter in the second imaging method, an imaging parameter for the second organ in the second imaging method.

The image processing apparatus may further include a reception unit configured to receive imaging range for the second organ.

The third feature identification unit may identify the settable range for the imaging parameter based on the first feature related to the first organ in the first image data.

The third feature identification unit may identify the settable range for the imaging parameter based on relative position of the first organ and the second organ in the first image data.

The image processing apparatus may further include a fourth feature identification unit configured to identify the second feature related to the second organ in the first image data.

The third feature identification unit may identify the settable range for the imaging parameter, based on the first feature related to the second organ in the first image data.

The image processing apparatus may further includes a fifth feature identification unit configured to identify position of a region of interest in the first organ in the first image data.

The third feature identification unit (135) may identify the settable range for the imaging parameter, based on position of the region of interest in the first organ in the first image data.

The image processing apparatus may further include a second acquisition unit configured to obtain constraint information related to a constraint on an apparatus used for the second imaging method.

The third feature identification unit may identify the settable range for the imaging parameter, based on the constraint information.

The estimation unit may identify a cross-section corresponding to imaging range for the second organ in the first image data, and accordingly estimate an imaging parameter corresponding to the second feature related to imaging range for the second organ.

The estimation unit may estimate a provisional imaging parameter, based on the first feature related to the first organ in the first image data and based on the second feature related to imaging range for the second organ.

When the provisional imaging parameter is within the settable range for the imaging parameter, the estimation unit may estimate the provisional parameter to be the imaging parameter, and when the provisional imaging parameter is outside the settable range for the imaging parameter, the estimation unit may exclude the provisional parameter and again perform operation of estimating the provisional imaging parameter.

The second feature identification unit may newly identify the second feature related to imaging range for the second organ in such a way that, when the provisional imaging parameter is outside the settable range for the imaging parameter, the second feature related to imaging range for the second organ becomes different than a previously-identified second feature related to imaging range for the second organ.

The estimation unit may estimate the provisional imaging parameter based on the newly-identified second feature related to imaging range for the second organ.

The first imaging method may indicate imaging performed using an X-ray CT apparatus, and the first image data may indicate CT image data obtained by an X-ray CT apparatus.

The second imaging method may indicate imaging according to transesophageal echocardiography, and the second image data may indicate ultrasound image data obtained during transesophageal echocardiography.

The first organ may indicate esophagus, and the second organ may indicate heart.

The image processing apparatus may further include an output unit configured to output the estimated imaging parameter to an image diagnostic apparatus that performs imaging according to the second imaging method.

According to a second aspect, an image processing system is provided, which includes the image processing apparatus and the image diagnostic apparatus as described above.

The image diagnostic apparatus includes a setting unit configured to set the imaging parameter output from the image processing apparatus as an imaging parameter for performing imaging according to the second imaging method, and an execution unit configured to perform imaging according to the second imaging method using the set imaging parameter.

According to a fourth aspect, an image processing method implemented in an image processing apparatus is provided.
The method includes obtaining first image data according to a first imaging method; identifying a first feature related to a first organ captured in the first image data; identifying, corresponding to imaging range for a second organ that is different than the first organ and that is captured in second image data obtained according to a second imaging method different than the first imaging method, a second feature related to imaging range for the second organ in the first image data; identifying a settable range for an imaging parameter in the second imaging method, and estimating based on the first feature related to the first organ in the first image data, the second feature related to imaging range for the second organ, and the settable range for an imaging parameter in the second imaging method, an imaging parameter for the second organ in the second imaging method.

According to a third aspect, an X-ray CT apparatus is provided, which includes a first acquisition unit configured to obtain first image data by performing imaging according to a first imaging method; a first feature identification unit configured to identify a first feature related to a first organ captured in the first image data; a second feature identification unit configured to identify, corresponding to imaging range for a second organ that is different than the first organ and that is captured in second-type image data obtained according to a second imaging method different than the first imaging method, a second feature related to imaging range for the second organ in the first image data; a third feature identification unit configured to identify a settable range for an imaging parameter in the second imaging method; and an estimation unit configured to estimate based on the first feature related to the first organ in the first image data, the second feature related to imaging range for the second organ, and the settable range for an imaging parameter in the second imaging method, an imaging parameter for the second organ in the second imaging method.

### BRIEF DESCRIPTION OF THE DRAWINGS

FIG. 1 is a block diagram illustrating an exemplary functional configuration of an image processing apparatus according to a first embodiment;
FIG. 2 is a schematic diagram illustrating an example of obtaining the features of a first organ in first image data according to the first embodiment;
FIG. 3 is a schematic diagram illustrating an example of image conversion in the first image data according to the first embodiment;
FIG. 4 is a diagram illustrating an example of a view name list that is displayed in a user interface according to the first embodiment;
FIG. 5 is a diagram illustrating an example of a view name list along with images according to the first embodiment;
FIGS. 6 to 10 are diagrams for explaining examples of imaging parameters according to the first embodiment;
FIGS. 11 to 15 are diagrams for explaining examples of an identification operation for identifying the settable range for an imaging parameter according to the first embodiment;
FIG. 16 is a schematic diagram illustrating an example of a user interface according to the first embodiment;
FIG. 17 is a flowchart for explaining an example of the operations performed by the image processing apparatus according to the first embodiment;
FIG. 18 is a block diagram illustrating an exemplary configuration of an image processing system according to a second embodiment;
FIG. 19 is a block diagram illustrating an exemplary configuration of an X-ray CT apparatus according to the second embodiment;
FIG. 20 is a block diagram illustrating an exemplary configuration of an ultrasound diagnostic apparatus according to the second embodiment;
FIGS. 21 to 23 are diagrams for explaining examples of an identification operation performed for identifying the settable range for an imaging parameter according to a first modification example;
FIG. 24 is a diagram illustrating an example of a display operation for displaying imaging parameters according to a fourth modification example;
FIG. 25 is a diagram illustrating an example of an identification operation for identifying the settable range for an imaging parameter according to a sixth modification example;
FIG. 26 is a diagram illustrating another example of the identification operation for identifying the settable range for an imaging parameter according to a sixth modification example;
FIG. 27 is a diagram for explaining an example of the identification operation for identifying the settable range for an imaging parameter according to the sixth modification example; and
FIG. 28 is a diagram for explaining an example of an imaging parameter according to a 1-th modification example.

### DETAILED DESCRIPTION

### First embodiment

Exemplary embodiments of an X-ray CT apparatus, an image processing apparatus, an image processing system and an image processing method are described below with reference to the accompanying drawings.

The image processing apparatus according to the embodiments is configured with a plurality of functional units installed as software in an independent computer, or installed in an equipment including a central processing unit (CPU) and a memory, or individually installed in a plurality of equipment. The functional units of the image processing apparatus that are stored in a memory are implemented when executed by a processor.

Alternatively, the functional units can be implemented using hardware in the form of circuitry capable of carrying out the functions of the image processing apparatus. The circuitry for operating the image processing apparatus can send and receive data or collect data via cyber means such as via the Internet. Meanwhile, the image processing apparatus according to the embodiments can also be directly attached to an image collection apparatus as a component.

The following explanation of the image processing apparatus and the image processing method is given with reference to an example of estimating the imaging parameters of transesophageal echocardiography (TEE) based on CT images.

For example, a first imaging method represents an imaging method for CT images, and a second imaging method represents an imaging method based on transesophageal echocardiography. Moreover, first image data represents CT images of the heart of the subject (hereinafter, heart CT images), and a first organ represents the esophagus of the subject. Furthermore, second image data represents medical images obtained using transesophageal echocardiography (i.e., ultrasound images of the heart of the subject), and a second organ represents the heart of the subject.

However, the embodiments are not limited to the example given above, and can obviously be applied in other types of image processing apparatuses and other image processing methods.

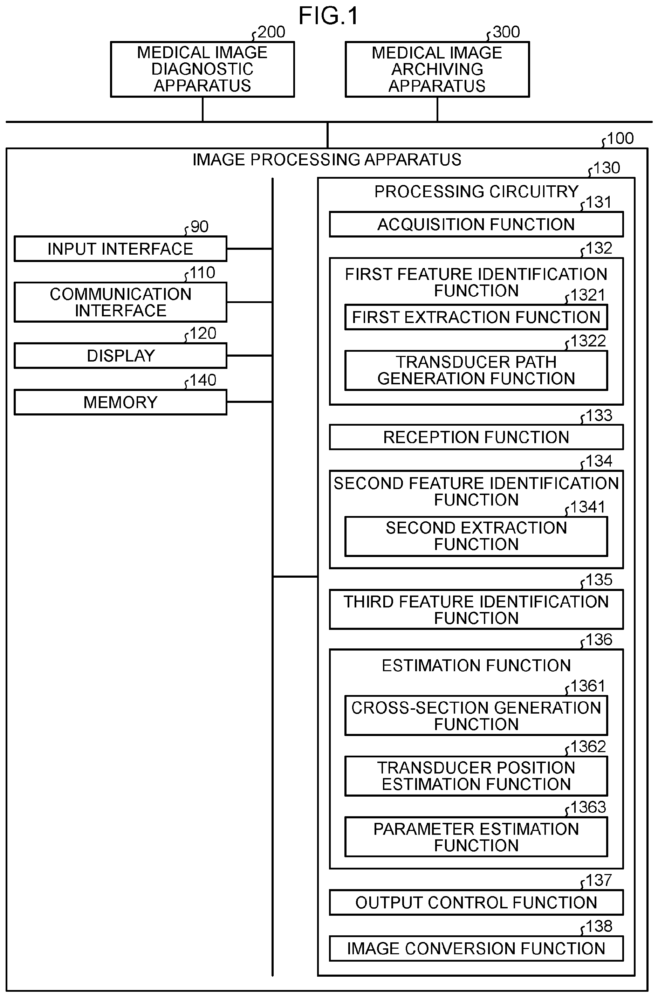

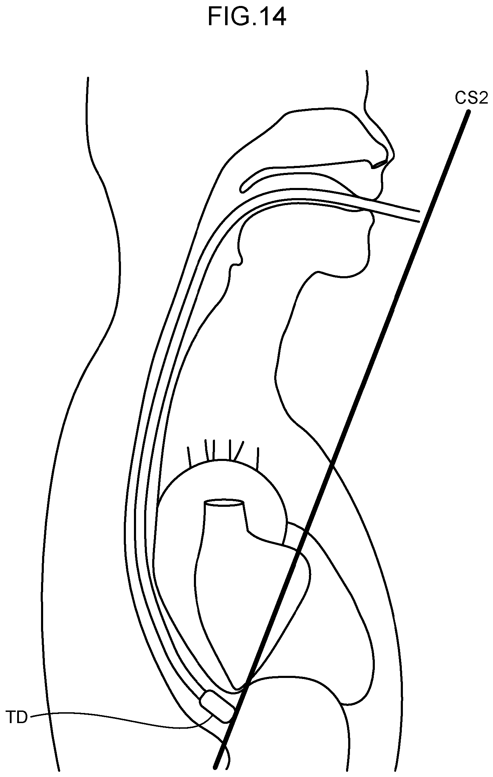

Regarding an image processing apparatus and an image processing method according to a first embodiment, the explanation is given below with reference FIGS. 1 to 17.

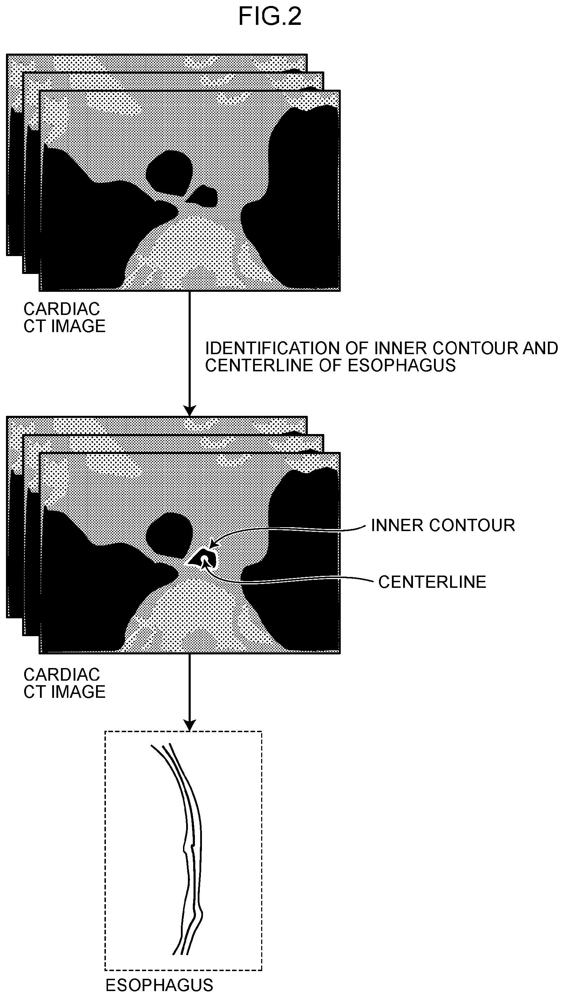

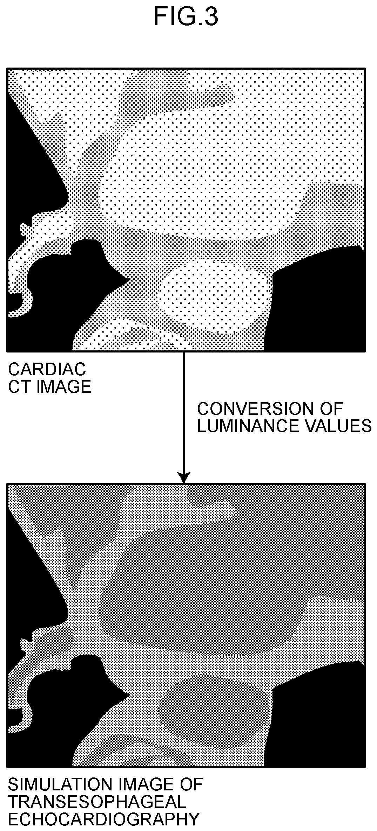

FIG. 1 is a block diagram illustrating an exemplary functional configuration of an image processing apparatus 100 according to the first embodiment. FIG. 2 is a schematic diagram illustrating an example of obtaining the features of the first organ in the first image data according to the first embodiment. FIG. 3 is a schematic diagram illustrating an example of image conversion in the first image data according to the first embodiment.

For example, as illustrated in FIG. 1, the image processing apparatus 100 according to the first embodiment is communicably connected to a medical image diagnostic apparatus 200 and a medical image archiving apparatus 300 via a network. Moreover, the network illustrated in FIG. 1 can also have various other types of apparatuses and systems connected thereto.

The medical image diagnostic apparatus 200 performs imaging with respect to the subject and generates a medical image. Then, the medical image diagnostic apparatus 200 sends the generated medical image to various apparatuses in the network. The medical image diagnostic apparatus 200 is, for example, an ultrasound diagnostic apparatus serving as an ultrasound system; or an X-ray CT apparatus (CT stands for Computer Tomography) or an MRI apparatus (MRI stands for Magnetic Resonance Imaging) that implements different modality than an ultrasound system.

The medical image archiving apparatus 300 archives a variety of medical images related to the subject. More particularly, the medical image archiving apparatus 300 receives a medical image from the medical image diagnostic apparatus 200 via the network, and archives that medical image in the memory provided therein.

The medical image archiving apparatus 300 is implemented using, for example, a computer equipment such as a server or a workstation. Alternatively, for example, the medical image archiving apparatus 300 is implemented using PACS (which stands for Picture Archiving and Communication System), and archives medical images in a format compatible to DICOM (Digital Imaging and Communications in Medicine).

Given below is the explanation of a configuration of the image processing apparatus 100 according to the first embodiment. As illustrated in FIG. 1, the image processing apparatus 100 includes an input interface 90, a communication interface 110, a display 120, a processing circuitry 130, and a memory 140.

The input interface 90 is implemented using, for example, a trackball used for various settings, or switch buttons, or a mouse, or a keyboard, or a touchpad enabling the input by touching on the operation screen, or a touchscreen configured by integrating a display screen and a touchpad, or a contactless input circuit in which an optical sensor is used, or a sound input circuit.

The input interface 90 is connected to the processing circuitry; and converts an input operation, which is received from an operator who is a user such as a doctor, into electrical signals and outputs the electrical signals to the processing circuitry 130. Meanwhile, with reference to FIG. 1, although the input interface 90 is disposed inside the image processing apparatus 100, it can alternatively be disposed outside the image processing apparatus 100.

The communication interface 110 is a network interface card (NIC) and communicates with other apparatuses. For example, the communication interface 110 is connected to the processing circuitry 130; and obtains medical images from the medical image diagnostic apparatus 200 or the medical image archiving apparatus 300, and outputs the medical images to the processing circuitry 130.

The display 120 is connected to the processing circuitry 130, and displays a variety of information and a variety of images that are output from the processing circuitry 130.

The display 120 is implemented using, for example, a liquid crystal monitor, a cathode ray tube (CRT) monitor, or a touch-sensitive panel. For example, the display 120 displays a graphical user interface (GUI) meant for receiving instructions from the operator, displays various display images, and displays various processing results obtained by the processing circuitry 130.

Meanwhile, with reference to FIG. 1, although the display 120 is disposed inside the image processing apparatus 100, it can alternatively be disposed outside the image processing apparatus 100.

The memory 140 is connected to the processing circuitry 130 and stores therein a variety of data. The memory 140 is implemented using, for example, a semiconductor memory device such as a random access memory (RAM) or a flash memory; or a hard disk; or an optical disk.

As an example, the memory 140 stores therein the data used in the processing performed by the processing circuitry 130, and the data generated as a result of the processing performed by the processing circuitry 130. More particularly, the memory 140 stores therein CT image data obtained from an X-ray CT apparatus (the medical image diagnostic apparatus 200) or the medical image archiving apparatus 300.

Moreover, the memory 140 stores therein computer program corresponding to the processing functions implemented by the processing circuitry 130.

Meanwhile, with reference to FIG. 1, although the memory 140 is disposed inside the image processing apparatus 100, it can alternatively be disposed outside the image processing apparatus 100.

The processing circuitry 130 comprehensively controls the image processing apparatus 100. For example, the processing circuitry 130 is implemented using a processor.

As illustrated in FIG. 1, the processing circuitry 130 includes an acquisition function 131, a first feature identification function 132, a reception function 133, a second feature identification function 134, a third feature identification function 135, an estimation function 136, an output control function 137, and an image conversion function 138.

The first feature identification function 132 includes a first extraction function 1321 and a transducer path generation function 1322. The second feature identification function 134 includes a second extraction function 1341. The estimation function 136 includes a cross-section generation function 1361, a transducer position estimation function 1362, and a parameter estimation function 1363.

The processing functions implemented by the acquisition function 131, the first feature identification function 132, the reception function 133, the second feature identification function 134, the third feature identification function 135, the estimation function 136, the output control function 137, and the image conversion function 138 representing the constituent elements of the processing circuitry 130 illustrated in FIG. 1 are stored as computer-executable programs in the memory 140 of the image processing apparatus 100.

The processing circuitry 130 is a processor that reads the computer programs from the memory 140 and executes them so as to implement the processing function corresponding to each function. In other words, upon reading the computer programs, the processing circuitry 130 becomes equipped with the functions specified in the processing circuitry 130 in FIG. 1.

For example, when the processor is a CPU, it reads the computer programs stored in the memory 140 and executes them to implement the functions. On the other hand, for example, when the processor is an ASIC, instead of storing the computer programs in the memory 140, they are directly embedded into the circuitry of the processor.

Meanwhile, a processor according to the first embodiment is not limited to be configurated as a singular circuitry. Alternatively, a plurality of independent circuitry can be combined to form a single processor in which the functions can be implemented. Still alternatively, a plurality of constituent elements illustrated in FIG. 1 can be integrated into a single processor in which the functions can be implemented.

Given below is the explanation about the processing details of the acquisition function 131, the first feature identification function 132, the reception function 133, the second feature identification function 134, the third feature identification function 135, the estimation function 136, the output control function 137, and the image conversion function 138 implemented by the processing circuitry 130.

The acquisition function 131 obtains the first image data corresponding to the first imaging method. More particularly, the acquisition function 131 obtains the first image data obtained as a result of imaging performed according to the first imaging method. The acquisition function 131 represents an example of a "first obtaining unit".

For example, before surgery, the acquisition function 131 obtains one or more cardiac phase images captured by a medical image collection apparatus according to computed tomography (CT), magnetic resonance (MR), or cone beam CT (CBCT).

In the first embodiment, the first imaging method is, for example, an imaging method for CT images; and the first image data is, for example, CT images of the heart of the subject. For example, from an X-ray CT device, which is an example of the medical image diagnostic apparatus 200, and via the input interface 90, the acquisition function 131 obtains medical images of the heart, which are captured according to the imaging method for CT images, as the first image data obtained according to the first imaging method (hereinafter, also referred to as cardiac CT images).

Alternatively, the acquisition function 131 can obtain the first image data from the medical image archiving apparatus 300. For example, the acquisition function 131 can monitor a PACS server, which represents an example of the medical image archiving apparatus 300, via a network and, when a new set of first image data is archived, can obtain that first image data.

The first feature identification function 132 identifies the features of the first organ captured in the first image data (cardiac CT images). The first feature identification function 132 represents an example of a "first feature identification function".

In the first embodiment, the first organ is, for example, the esophagus. More particularly, as illustrated from the upper diagram to the middle diagram in FIG. 2, from the cardiac CT images obtained by the acquisition function 131, the first feature identification function 132 identifies the features related to the esophagus in those cardiac CT images.

The features related to the esophagus indicate, for example, the inner contour and the centerline of the esophagus. Meanwhile, as explained earlier, the first feature identification function 132 includes the first extraction function 1321 and the transducer path generation function 1322.

Moreover, based on the features related to the first organ in the cardiac CT images as identified by the first feature identification function 132, the first extraction function 1321 extracts the region indicating the esophagus in the cardiac CT images. That is, the first extraction function 1321 obtains coordinate information of the pixels representing the esophagus in the cardiac CT images.

More particularly, the first extraction function 1321 receives, from the operator, a specification input about the region indicating the esophagus and accordingly identifies the region indicating the esophagus in the medical images. Then, based on the identified region indicating the esophagus, the first extraction function 1321 obtains the coordinate information of each pixel corresponding to that region.

Alternatively, the first extraction function 1321 can implement a known region extraction method to identify the region indicating the esophagus based on the anatomical structure extracted from the cardiac CT images. Examples of the known region extraction method include Otsu's method based on CT values, the region growing method, the snake method, the graph cut method, and the mean shift clustering.

Still alternatively, the first extraction function 1321 can identify the region indicating the esophagus using a shape model generated according to a known machine learning technology (including deep learning).

In that case, for example, with respect to a cardiac CT image obtained by the acquisition function 131, the first extraction function 1321 applies a shape model capable of extracting the coordinate information of a plurality of pixels corresponding to the region indicating the esophagus in the cardiac CT image. Then, based on the extraction result obtained as a result of applying the shape model, the first extraction function 1321 identifies the region indicating the esophagus.

In this case, the shape model is, for example, a trained model that, using a known machine learning technology, is trained in the relationship between the cardiac CT images, in which the region indicating the esophagus is rendered and which are treated as the input-side teacher data, and the coordinate information of a plurality of pixels, which corresponds to the region indicating the esophagus in the cardiac CT images and which is treated as the output-side teacher data, according to the dataset including the two types of teacher data.

Meanwhile, instead of extracting the entire region of the esophagus, the first extraction function 1321 can extract only some part of that region. For example, since the esophagus has a luminal structure, the first extraction function 1321 can extract only the inner contour and the centerline of the esophagus.

In this way, according to a known method, the first extraction function 1321 extracts, from a cardiac CT image, the esophagus (the inner contour and the centerline of the esophagus) as illustrated in the lower drawing in FIG. 2.

Based on the first organ (the esophagus of the subject) extracted by the first extraction function 1321, the transducer path generation function 1322 generates, in the first image data (a cardiac CT image), a path for a transducer meant for taking images of the second organ according to the second imaging method.

In the first embodiment, the second imaging method is, for example, transesophageal echocardiography (TEE), and the second organ is the heart of the subject.

In that case, based on the features related to the esophagus as obtained by the first extraction function 1321, the transducer path generation function 1322 generates, in a cardiac CT image, a path for a transducer meant for taking images of the heart according to transesophageal echocardiography.

That is, based on the features related to the esophagus as obtained by the first extraction function 1321, the transducer path generation function 1322 generates, as a transducer path, a path on which a transducer can move inside the esophagus.

A transducer is a medical equipment used when an ultrasound system takes medical images of a subject. A transducer converts electrical signals of the ultrasound system into ultrasound waves; and, when the ultrasound waves get reflected from the inside of the subject, receives the reflected ultrasound waves and converts them into electrical signals, and outputs the electrical signals to the ultrasound system. As a result, medical images are formed.

The image conversion function 138 processes the luminance values of the first image data in such a way that the luminance values based on the first imaging method are converted into the luminance values based on the second imaging method.

In a CT image and an ultrasound image, identical tissue gets displayed with different luminance values.

For example, in a cardiac CT image, the four chambers of the heart (the right atrium, the right ventricle, the left atrium, and the left ventricle) are sometimes displayed with high a luminance value (i.e., sometimes appear almost white) due to the effect of a contrast medium. However, in a medical image obtained by transesophageal echocardiography (i.e., in an ultrasound image), the four chambers of the heart are sometimes displayed with a low luminance value (i.e., sometimes appear almost black).

With reference to the upper and lower drawings in FIG. 3, the image conversion function 138 processes the luminance values in a cardiac CT image and converts the luminance value of the tissue of each organ in the cardiac CT image based on the imaging method for CT images into the luminance value in transesophageal echocardiography based on the imaging method for ultrasound images.

As a result, in the first embodiment, using a cardiac CT image (i.e., a CT image), it becomes possible to estimate (simulate) a medical image taken according to transesophageal echocardiography (i.e., an ultrasound image).

The reception function receives the imaging range that corresponds to the second image data obtained by performing imaging according to the second imaging method, which is different than the first imaging method, and that is related to the second organ, which is different than the first organ. The reception function 133 represents an example of a "receiving unit".

As explained above, for example, the first imaging method represents the imaging method for CT images, and the second imaging method represents imaging method based on transesophageal echocardiography. Moreover, the first organ represents the esophagus of the subject, and the second organ represents the heart of the subject.

For example, the operator uses a user interface 71 (explained later) and specifies the imaging range for the heart during transesophageal echocardiography. More particularly, from a list displayed in the user interface 71 (explained later), the operator selects the view name corresponding to the imaging range for the heart during transesophageal echocardiography (hereinafter, simply referred to as the imaging range for transesophageal echocardiography).

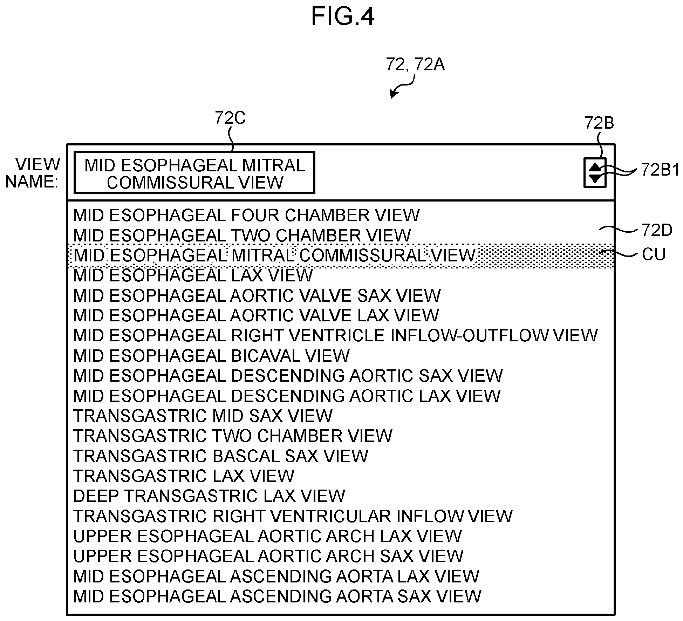

In FIG. 4 is illustrated an example of a view name list 72D that is displayed in the user interface 71 according to the first embodiment. In the view name list 72D is displayed a list of view names that can be selected by the operator.

In the example illustrated in FIG. 4, in the view name list 72D, the following 20 view names are displayed: Mid Esophageal Four Chamber View, Mid Esophageal Two Chamber View, Mid Esophageal Mitral Commissural View, Mid Esophageal LAX View, Mid Esophageal Aortic Valve SAX View, Mid Esophageal Aortic Valve LAX View, Mid Esophageal Right Ventricle Inflow-Outflow View, Mid Esophageal Bicaval View, Mid Esophageal Descending Aortic SAX View, Mid Esophageal Descending Aortic LAX View, Transgastric Mid SAX View, Transgastric Two Chamber View, Transgastric Bascal SAX View, Transgastric LAX View, Deep Transgastric LAX View, Transgastric Right Ventricular Inflow View, Upper Esophageal Aortic Arch LAX View, Upper Esophageal Aortic Arch SAX View, Mid Esophageal Ascending Aorta LAX View, and Mid Esophageal Ascending Aorta SAX View.

Moreover, in the view name list 72D, a cursor CU is displayed for enabling the operator to select a view name. For example, the cursor CU moves up and down according to a button 72B1 on the right side 72B of a display area 72A (explained later). The view name selected using the cursor CU gets displayed in a middle portion 72C of the display area 72 of a display area 72 (explained later).

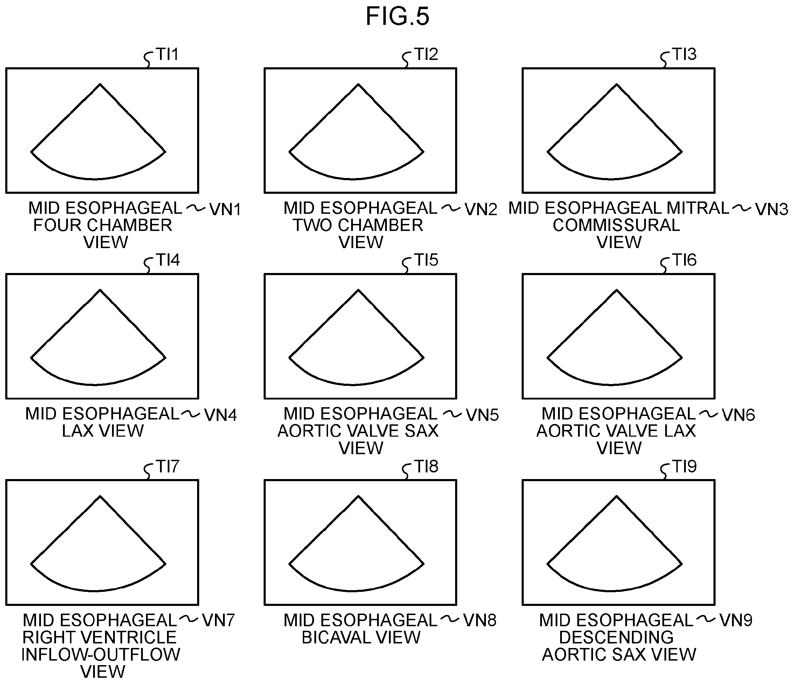

Meanwhile, the view name list 72D can include images too. In FIG. 5 is illustrated an example of a view name list 72D1 along with images.

In the view name list 72D1 illustrated in FIG. 5, view name display fields VN (VN1 to VN9) and typical images TI (TI1 to TI19) are displayed. Each view name VN represents a display field for displaying the information indicating the view name.

Each typical image display fields TI represents a display field in which a typical image is displayed that is obtained as a result of performing transesophageal echocardiography for the corresponding view name. As an example, in the typical image display field T11, a typical image is displayed for the case in which the imaging range for transesophageal echocardiography corresponds to a view name VN1 "Mid Esophageal Four Chamber View".

The image displayed in the typical image display field T1 can be a video too.

Regarding the information related to the imaging range for transesophageal echocardiography, the operator can select either a single set of information or a plurality of sets of information.

In this way, as the imaging range for capturing the second organ according to the second imaging method, the reception function 133 receives the imaging range specified by the operator using the user interface 71 (explained later).

The second feature identification function 134 identifies the features related to the second organ in the first image data (a cardiac CT image). In that case, the second feature identification function 134 represents an example of a "fourth feature identifying unit".

In the first embodiment, the second organ is, for example, the heart. More particularly, from the cardiac CT image obtained by the acquisition function 131, the second feature identification function 134 identifies the features related to the heart captured in that cardiac CT image.

Herein, the features related to the heart indicate, for example, the region indicating the entire heart and the important structures therein. Meanwhile, as explained above, the second feature identification function 134 includes the second extraction function 1341.

Moreover, based on the identified features related to the heart captured in the cardiac CT image, the second extraction function 1341 extracts the region indicating the heart in the cardiac CT image. That is, the second extraction function 1341 obtains the coordinate information of the pixels indicating the heart in the cardiac CT image.

More particularly, the second extraction function 1341 receives, from the operator, a specification input about the region indicating the entire heart and the important structures therein and accordingly identifies the region indicating the entire heart and the important structures therein. Then based on the identified region indicating the entire heart and the important structures therein, the second extraction function 1341 obtains the coordinate information about each pixel corresponding to that region.

Alternatively, the second extraction function 1341 can identify the region indicating the entire heart and the important structures therein based on the anatomical structure extracted from the cardiac CT images according to a known region extraction method. Still alternatively, the second extraction function 1341 can identify the region indicating the entire heart and the important structures therein using a shape model generated according to a known machine learning technology.

In that case, for example, with respect to the cardiac CT image obtained by the acquisition function 131, the second extraction function 1341 applies a shape model capable of extracting the coordinate information of a plurality of pixels corresponding to the region indicating the entire heart and the important structures therein as captured in the cardiac CT image. Then, based on the extraction result obtained as a result of applying the shape model, the second extraction function 1341 identifies the region indicating the entire heart and the important structures therein.

In this case, the shape model is, for example, a trained model that, using a known machine learning technology, is trained in the relationship between the cardiac CT images, in which the region indicating the entire heart and the important structures therein is rendered and which are treated as the input-side teacher data, and the coordinate information of a plurality of pixels, which corresponds to the region indicating the entire heart and the important structures therein as captured in the cardiac CT images and which is treated as the output-side teacher data, according to the dataset including the two types of teacher data.

Meanwhile, the second feature identification function 134 can identify the features related only to the imaging range for the second organ (the heart of the subject) in the first image data (the cardiac CT image). In that case, the second feature identification function 134 represents an example of a "second feature identifying unit".

For example, based on the imaging range for the second organ (the heart of the subject) as received by the reception function 133, the second feature identification function 134 can identify the features related to the imaging range for the heart of the subject from a template stored in the memory 140.

When a template for the CT images is stored in the memory 140, that template serves as the template for the medical images taken during transesophageal echocardiography.

For example, when a template including the features related to the imaging range for the second organ (the heart of the subject) in the first image data (a cardiac CT image) is stored in the memory 140, that template serves as, for example, the template for the medical images (ultrasound images) taken during transesophageal echocardiography.

The template includes the information indicating the view name, the information indicating the anatomical features, and the information indicating the algorithm sequence; and that information is stored in advance in a corresponding manner in the memory 140.

The information indicating the anatomical features indicates the anatomical features corresponding to the view name. Examples of the anatomical features include the mitral center, the left ventricle (LC) apex, the tricuspid center, the mitral center, the anterolateral (AL) commissural, the posteromedial (PM) commissural, and the minimum square sum of mitral valve.

The information indicating the algorithm sequence indicates the algorithm sequence meant for extracting the anatomical features. Examples of the algorithm sequence include chamber segmentation, mitral segmentation, LV apex detection, and tricuspid segmentation.

In the case mentioned above, based on the correspondence relationship between the view name and the anatomical feature in the template, the second feature identification function 134 can identify the anatomical features related to the heart in the imaging range for the heart in the cardiac CT image. That is done for estimating (simulating) the medical images which would be taken during transesophageal echocardiography.

Moreover, based on the correspondence relationship between the view name and the algorithm sequence in the template, the second feature identification function 134 can identify the algorithm sequence corresponding to the anatomical features related to the imaging range for the heart in the cardiac CT image. That is done to extract the anatomical features in the cardiac CT image.

Meanwhile, a shape model corresponding to each view name can be kept ready, and the second feature identification function 134 (the second extraction function 1341) can identify/extract the features related to the imaging region for the second organ (the heart of the subject) corresponding to the concerned view name.

The third feature identification function 135 identifies an imaging parameter settable range within which the imaging parameters in the second imaging method (transesophageal echocardiography) can be set. The third feature identification function 135 represents an example of a "third feature identifying unit". The imaging parameter settable range indicates the range of the actually-capturable imaging parameters in the second imaging method.

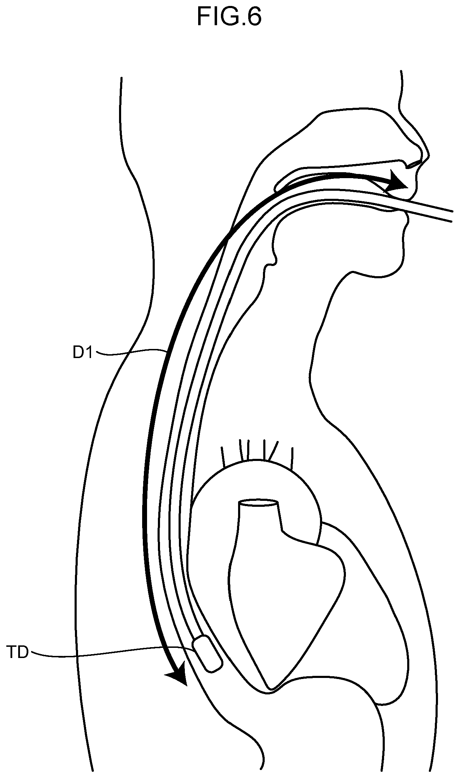

Herein, the imaging parameters represent the parameters estimated by the estimation function 136 (explained later) and imply the imaging conditions during transesophageal echocardiography. Regarding the imaging parameters according to the first embodiment, the explanation is given below with reference to FIGS. 6 to 10. FIGS. 6 to 10 are diagrams for explaining an example of the imaging parameters according to the first embodiment.

In the first embodiment, the imaging parameters include distance D1, sector rotation A1 (rotation), front-back direction rotation angle A1 (anteflex/retroflex), left-right direction rotation angle A3 (left/right flexion), sector depth D2 (scan depth), and section angle A4 (scan range).

As illustrated in FIG. 6, the distance D1 (hereinafter, also simply referred to as D1) indicates the distance from the mouth of the subject to the leading end of a transducer TD during transesophageal echocardiography. In the first embodiment, the distance D1 is expressed in the unit of [cm].

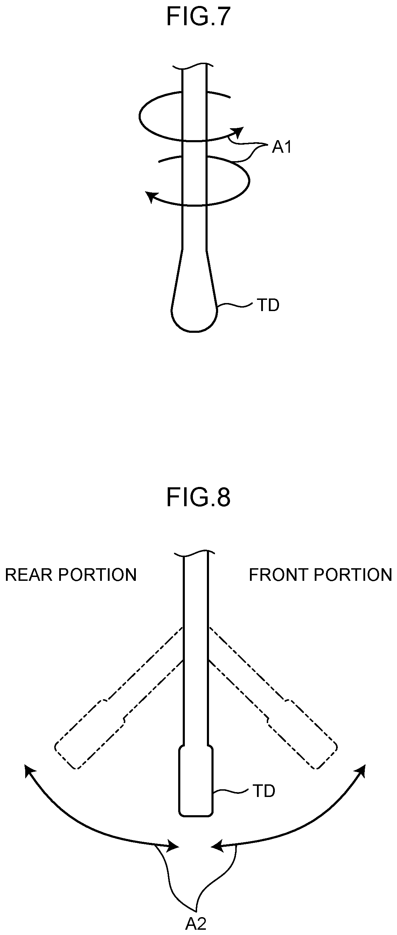

As illustrated in FIG. 7, the sector direction A1 (hereinafter, also simply referred to as A1) indicates the angle of rotation of the transducer TD in the axial direction during transesophageal echocardiography. In the first embodiment, the sector direction A1 is expressed in the unit of [°] (degree) according to the degree measure.

As illustrated in FIG. 8, the front-back direction rotation angle A2 (hereinafter, also simply referred to as A2) indicates the angle of rotation of the transducer TD in the front-back direction during transesophageal echocardiography. In the first embodiment, in the default state, the front-back direction rotation angle A2 is assumed to be equal to 0°. From the default state, the angles of rotation in the front direction are expressed as positive angles, and the angles of rotation in the back direction are expressed as negative angles. In the first embodiment, the front-back direction rotation angle A2 is expressed in the unit of [°] (degree) according to the degree measure.

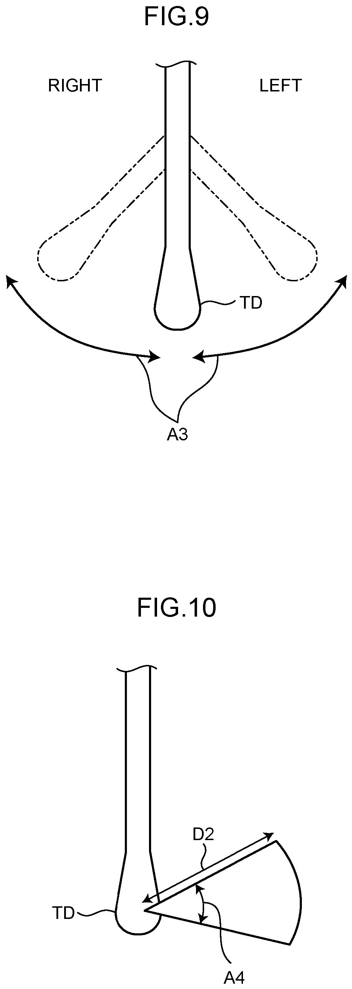

As illustrated in FIG. 9, the left-right direction rotation angle A3 (hereinafter, also simply referred to as A3) indicates the angle of rotation of the transducer TD in the left-right direction during transesophageal echocardiography. In the first embodiment, in the default state, the left-right direction rotation angle A3 is assumed to be equal to 0°. From the default state, the angles of rotation in the left direction are expressed as positive angles, and the angles of rotation in the right direction are expressed as negative angles. In the first embodiment, the front-back direction rotation angle A2 is expressed in the unit of [°] (degree) according to the degree measure. In the first embodiment, the left-right direction rotation angle A23 is expressed in the unit of [°] (degree) according to the degree measure.

As illustrated in FIG. 10, the sector depth D2 (hereinafter, also simply referred to as D2) and the section angle A4 (hereinafter, also simply referred to as A4) respectively indicate the depth and the angle of the scan range during transesophageal echocardiography. In the first embodiment, the section depth D2 is expressed in the unit of [cm], and the section angle A4 is expressed in the unit of [°] (degree) according to the degree measure.

Meanwhile, imaging parameters mentioned above are only exemplary, and other imaging parameters can also be included.

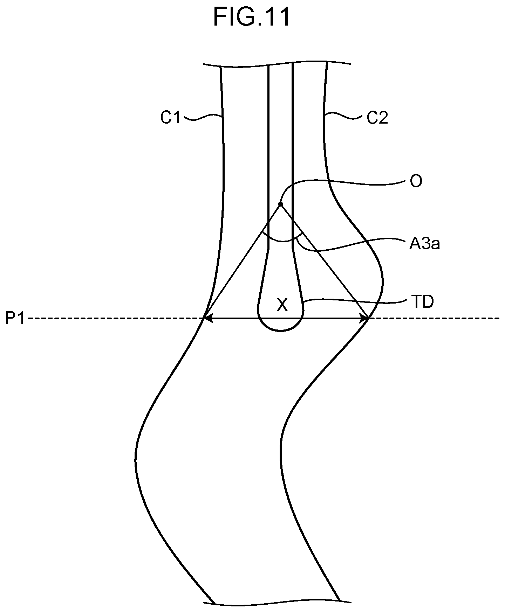

Explained below with reference to FIGS. 11 to 15 is an identification operation performed by the third feature identification function 135 for identifying the settable range for an imaging parameter. FIGS. 11 to 15 are diagrams for explaining an example of the identification operation for identifying the settable range for an imaging parameter according to the first embodiment.

For example, based on the anatomical structure of the esophagus as identified by the first feature identification function 132, the third feature identification function 135 identifies the imaging parameter settable range.

More particularly, the third feature identification function 135 calculates the length of the curve of the centerline of the esophagus extracted by the first extraction function 1321. The third feature identification function 135 identifies the maximum value of the calculated length of the curve of the centerline of the esophagus as the settable range for the distance D1.

Moreover, for example, based on the diameter of the esophagus at each position, the third feature identification function 135 identifies the settable range for the front-back direction rotation angle A2 and for the left-right direction rotation angle A3.

With reference to FIG. 11, curved lines C1 and C2 represent the inner contour of the esophagus. As illustrated in FIG. 11, when X represents the diameter in the left-right direction of the esophagus at a position P1, the third feature identification function 135 identifies an angle A3a, at which the transducer TD positioned on the centerline of the esophagus is movable in the left-right direction, as the settable range for the left-right direction rotation angle A3.

Meanwhile, although not illustrated in the drawings, in an identical manner, based on the diameter in the front-back direction at the position P1 of the esophagus, the third feature identification function 135 identifies the angle at which the transducer TD positioned on the centerline of the esophagus is movable in the front-back direction as the settable range for the front-back direction rotation angle A2. Moreover, the front-back direction rotation angle A2 or the left-right direction rotation angle A3 is calculated from the relationship between the position of the inner contour of the esophagus corresponding to the position P1 thereof and a start point O of movability of the transducer TD. Alternatively, the front-back direction rotation angle A2 and/or the left-right direction rotation angle A3 corresponding to a plurality of angles of the sector rotation A1 can be calculated from the relationship between the closed curve, which indicates the inner contour of the esophagus on the two-dimensional cross-sectional surface corresponding to the position P1 of the esophagus, and the start point O of movability of the transducer TD.

Meanwhile, at each position of the esophagus, when the transducer TD is positioned parallel to the esophagus, if the sector rotation A1 and the front-back direction rotation angle A2 are varied, the third feature identification function 135 can treat the range in which there is no interference between the esophagus wall and the transducer TD as the settable range for the front-back direction rotation angle A2 and the left-right direction rotation angle A3.
That is, the third feature identification function 135 can calculate the settable range for the front-back direction rotation angle A2 and the left-right direction rotation angle A3 from the diameter at each position of the esophagus and the size of the transducer TD.

Meanwhile, it is a known fact that the esophagus is flexible to a certain extent. Hence, as the settable range for the front-back direction rotation angle A2 and the left-right direction rotation angle A3, the third feature identification function 135 can set the angle obtained when a predetermined angle (margin) representing the non-interfering range is added to the angle calculated from the diameter at each position of the esophagus and the size of the transducer TD.

However, if the settable range for the front-back direction rotation angle A2 and the left-right direction rotation angle A3 is set at each position of the esophagus, the complexity increases. For that reason, the third feature identification function 135 can identify the maximum value and the minimum value of the possible angles at each position of the esophagus, and can identify the range between the maximum value and the minimum value as the settable range for the front-back direction rotation angle A2 and the left-right direction rotation angle A3.

Moreover, for example, the third feature identification function 135 can identify the settable range for the sector rotation A1 based on the positional relationship between the structure of the esophagus identified by the first feature identification function 132 and structure of the heart identified by the second feature identification function 134.

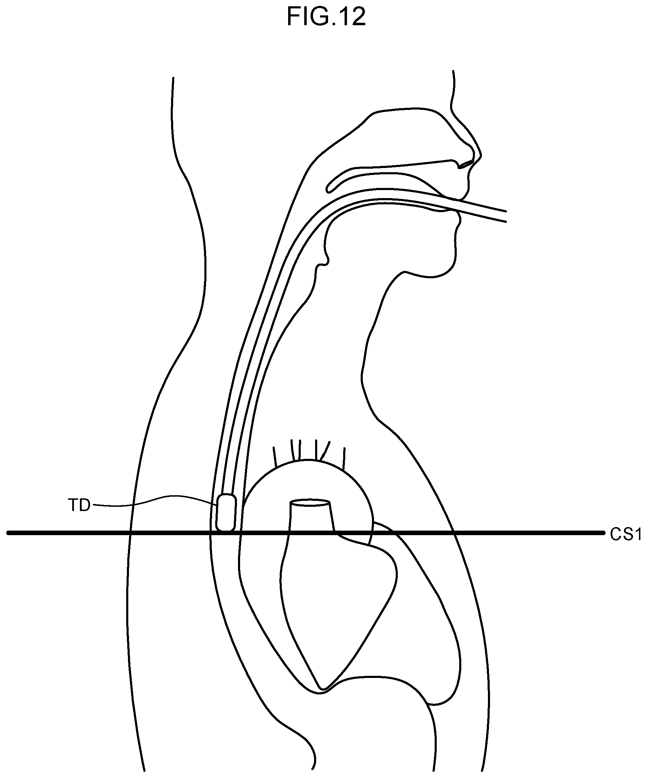

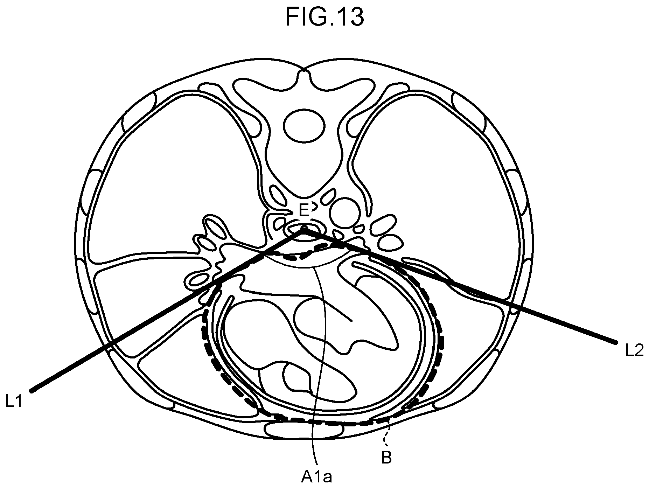

As an example, as illustrated in FIG. 12, the third feature identification function 135 sets a cross-section CS1 at each position of the esophagus. Then, as illustrated in FIG. 13, the third feature identification function 135 identifies, as the settable range for the sector rotation A1, an angle Ala formed by two tangent lines L1 and L2 that can be drawn from a position E at the center of the esophagus in the cross-section CS1 toward a boundary position B of the heart on the cross section CS.

Meanwhile, in the case of identifying the settable range for the sector rotation A1, the cross-section set by the third feature identification function 135 can be a horizontal cross-section as illustrated in FIG. 12 or can be a cross-section set based on the holding angle of transducer TD (for example, vertically) inferred from the shape of the esophagus (when the transducer TD is placed parallel to the shape of the esophagus).

Meanwhile, if the settable range for the sector rotation A1 is set at each position of the esophagus, the complexity increases. For that reason, the third feature identification function 135 can identify the maximum value and the minimum value of the possible angles at each position of the esophagus, and can identify the range between the maximum value and the minimum value as the settable range for the sector rotation A1.

Moreover, for example, the third feature identification function 135 identifies the settable range for the sector depth D2 and the section angle A4 based on the positional relationship between the structure of the esophagus identified by the first feature identification function 132 and the structure of the heart identified by the second feature identification function 134.

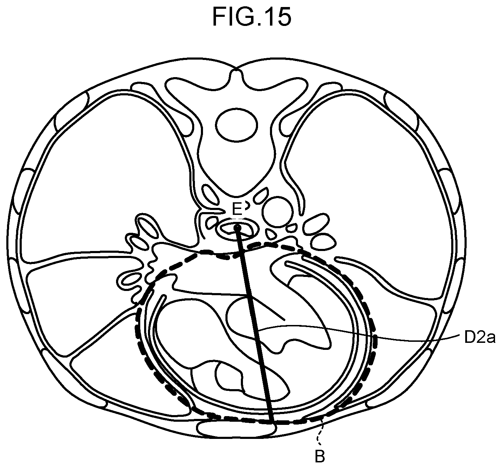

As an example, as illustrated in FIG. 15, in the cross-section CS1 illustrated in FIG. 12 and set at each position of the esophagus, the third feature identification function 135 calculates a maximum distance D2a from the position E at the center of the esophagus to the boundary position B of the heart in the cross-section CS1. Then, based on the maximum distance D2a, the third feature identification function 135 identifies the settable range for the sector depth D2.

At that time, the third feature identification function either can identify the calculated maximum distance D2a to be the settable range for the sector depth D2, or can identify the settable range for the sector depth D2 to be the maximum distance D2 with some margin.

Moreover, the third feature identification function 135 identifies the settable range for section angle A4 based on the same identification method as the identification method implemented to identified the settable range for the sector rotation A1.

Meanwhile, as the settable range for the sector depth D2 and the section angle A4, the third feature identification function 135 can set such numerical values which are settable as the constraints for a transesophageal echocardiography apparatus. In that case, information indicating the settable range for the sector depth D2 and the section angle A4 of the transducer TD can be stored in the memory 140. Meanwhile, when a plurality of types of transesophageal echocardiography apparatuses is available, information indicating the settable range for the sector depth D2 and the section angle A4 of the transducer TD can be stored separately for each type of transesophageal echocardiography apparatus.

In the case mentioned above, the third feature identification function 135 obtains, from the memory 140, the numerical values that can be set as the constraints for a transesophageal echocardiography apparatus and identifies those numerical values as the settable range for the sector depth D2 and the section angle A4. Hence, in that case, it can be said that the third feature identification function 135 represents an example of a "second obtaining unit".

Meanwhile, the method for identifying the settable range for imaging parameters during transesophageal echocardiography as explained above is only exemplary. Thus, the method for identifying the settable range for imaging parameters is not limited to the method explained above. In essence, as long as the settable range for imaging parameters is set based on the structure identified either by the first feature identification function 132 or by the second feature identification function 134 or is set based on the constraints on the structure of a transesophageal echocardiography apparatus, the identification can be performed according to any method.

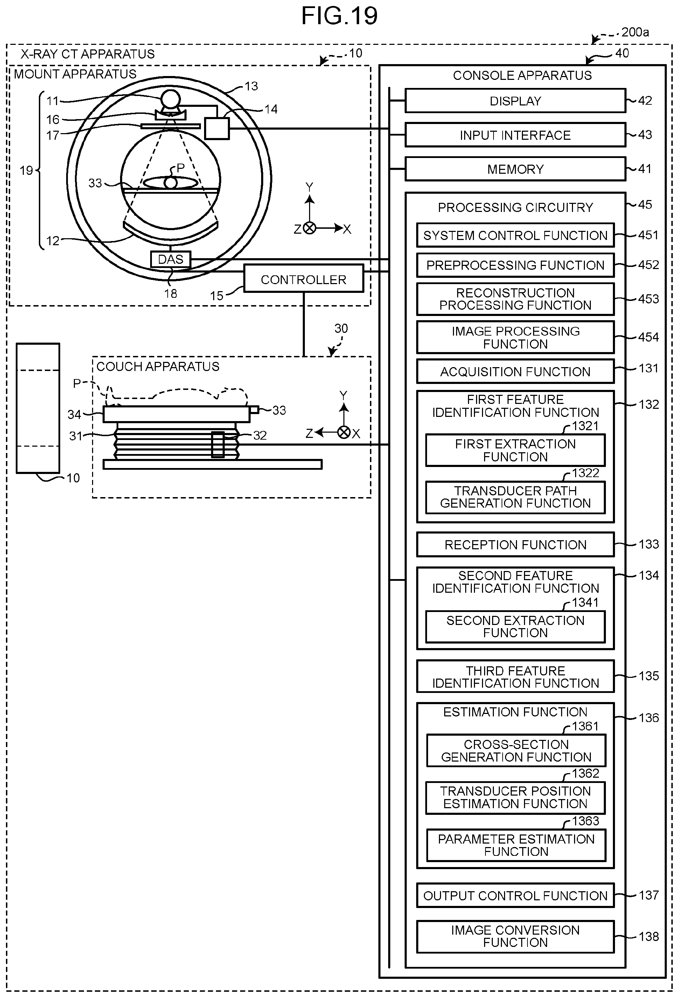

Returning to the explanation with reference to FIG. 19, based on the features (the inner contour and the center line) of the first organ (the esophagus of the subject) in the first image data (a cardiac CT image), based on the features related to the imaging range for the second organ (the heart of the subject), and based on the settable range for imaging parameters in the second imaging method; the estimation function 136 estimates the imaging parameters at the time of capturing the imaging range for the second organ according to the second imaging method. The estimation function 136 represents an example of an "estimating unit".

As explained earlier, the estimation function 136 includes the cross-section generation function 1361, the transducer position estimation function 1362, and the parameter estimation function 1363.

The cross-section generation function 1361 identifies the positions that correspond to the imaging range for transesophageal echocardiography as received by the reception function 133 from the cardiac CT image obtained by the acquisition function 131.

For example, the cross-section generation function 1361 identifies the positions that correspond to the imaging range for transesophageal echocardiography as received using a learning model generated based on a known machine learning technology.

In this case, the learning model is, for example, a trained model that, using a known machine learning technology, is trained in the relationship between imaging range information, which indicates the imaging range for transesophageal echocardiography and which is treated as the input-side teacher data, and a plurality of important positions (landmarks), which is present in the structure of the heart in a large number of cardiac CT images prepared in advance and which is treated as the output-side teacher data, according to the dataset including the two types of teacher data.

In that case, regarding each imaging range during transesophageal echocardiography that is likely to be received by the reception function 133, the cross-section generation function 1361 sets in advance the anatomical positions representing the landmark necessary for the identification of the imaging range, and identifies those anatomical positions.

Herein, in order to identify a single cross-section, it is sufficient to know at least three landmarks. That is, if three landmarks corresponding to each imaging range are set in advance and if a learning model is generated that is trained while using the positions of the landmark groups as the output-side teacher data, the cross-section generation function 1361 can use the learning model and identify the cross-section corresponding to the imaging range for transesophageal echocardiography as received by the reception function 133.

In the case explained above, when 20 imaging ranges are likely to be received, the maximum number of landmarks to be set in advance is equal to 60. If there is repetition of the landmarks, their number becomes smaller than 60.

As another example, the learning model can be trained in the relationship between the information indicating the imaging range for transesophageal echocardiography, which is treated as the input-side teacher data, and coordinate information of a plurality of pixels, which corresponds to the imaging range for transesophageal echocardiography in a large number of cardiac CT images prepared in advance and which is treated as the output-side teacher data, according to the dataset including the two types of teacher data.

As another example, the learning model can be trained in the relationship between the information indicating the imaging range for transesophageal echocardiography, which is treated as the input-side teacher data, and cross-sectional images, which correspond to the imaging range for transesophageal echocardiography in a large number of cardiac CT images prepared in advance and which are treated as the output-side teacher data, according to the dataset including the two types of teacher data.

Moreover, in the first embodiment, at the time of identifying the positions corresponding to the imaging range for transesophageal echocardiography, the cross-section generation function 1361 limits the search range based on the settable range for imaging parameters that is used in the case of performing transesophageal echocardiography and that is identified by the third feature identification function 135; and, only within that search range, identifies the position corresponding to the imaging range for the heart.

For example, the cross-section generation function 1361 generates such a cardiac CT image in which, based on the settable range for the distance D1, the region having the coordinates in the body axis direction of the cardiac CT image (i.e., the Z coordinates) positioned outside the D1-based image range is deleted. Then, the cross-section generation function 1361 treats the generated cardiac CT image as the input data for a learning model.

Alternatively, the cross-section generation function 1361 can uses the settable range for some other imaging parameter other than the distance D1, and accordingly limit the data to be input to a learning model.

In this way, as a result of limiting the data to be input to a learning model, such imaging parameters which are outside the settable range of the imaging parameters estimated by the estimation function 136 in the case of performing transesophageal echocardiography are no longer calculated.

Meanwhile, when a plurality of imaging ranges for transesophageal echocardiography is received by the reception function 133, with respect to each imaging range for transesophageal echocardiography, the cross-section generation function 1361 identifies the corresponding positions in the cardiac CT image.

Moreover, based on the identified positions (the cross-section) corresponding to the imaging range for transesophageal echocardiography as received by the reception function 133, the cross-section generation function 1361 generates an MPR cross-section (MPR stands for Multi Planar Reconstruction) corresponding to that imaging range. The MPR cross-section represents, for example, the cross-section used at the time of estimating (simulating) a medical image, which would be taken during transesophageal echocardiography, using the cardiac CT image.

Meanwhile, in a cardiac CT image, when the anatomical features related to the heart in the imaging range for the heart are identified by the second feature identification function 134, the cross-section generation function 1361 can generate an MPR cross-section based on the anatomical features.

Subsequently, the transducer position estimation function 1362 estimates the position of the transducer TD based on the features related to the first organ as identified by the first feature identification function 132 and the cross-section generated by the cross-section generation function 1361.

More particularly, as explained earlier, the first organ is the esophagus of the subject; the features related to the first organ include the inner contour and the centerline of the esophagus; and the generates cross-section is an MPR cross-section. For example, the transducer position estimation function 1362 estimates, as the position of the transducer TD, the point of intersection between the centerline of the esophagus as identified by the first feature identification function 132 and the MPR cross-section generated by the cross-section generation function 1361.

Subsequently, according to the position of the mouth of the subject in the cardiac CT image and according to the position of the transducer TD in the MPR cross-section as estimated by the transducer position estimation function 1362, the parameter estimation function 1363 calculates (estimates) the distance D1 as the imaging parameter.

Then, based on the estimated position of the transducer TD and based on the positional relationship between the esophagus and the heart in the MPR cross-section, the parameter estimation function 1363 estimates the angles A1 to A4 and the sector depth D2 as the imaging parameters.

For example, according to the positional relationship between the position of the transducer TD, the esophagus, and the heart in the MPR cross-section estimated by the transducer position estimation function 1362, the parameter estimation function 1363 estimates the angles A1 to A3 in such a way that a cross-section corresponding to the MPR cross-section can be captured during transesophageal echocardiography.

Moreover, for example, based on the three landmark identified by the cross-section generation function 1361, the parameter estimation function 1363 estimates the imaging parameters such as the sector depth D2 and the section angle A4 in such a way that the sector includes all of the three landmarks and that the area of the sector becomes the smallest.

More particularly, the parameter estimation function 1363 estimates the sector depth D2 and the section angle A4 at the time of performing transesophageal echocardiography. In that case, during the actual process of transesophageal echocardiography, smaller the area of the sector, the higher is the image quality. On the other hand, greater the area of the sector, the lower is the image quality. Meanwhile, as a condition, it is required to set the sector depth D2 and the section angle A4 in such a way that the anatomical positions identified by the cross-section generation function 1361 can be captured.

Herein, as an example, the parameter estimation function 1363 implements a method such as the loss function that indicates the difference between the output of the "target" and the "actual". For example, in a neural network, at the time of deciding on the most suitable parameters for enabling accurate inference, it is the loss function that serves as the benchmark for approaching the most suitable parameters.

Hence, at the transducer position in the MPR cross-section, using the loss function which satisfies the abovementioned condition and by which the loss increase in proportion to the increase in the area of the sector, the parameter estimation function 1363 estimates the sector depth D2 and the section angle A4 as the parameters thereof.

Returning to the explanation with reference to FIG. 19, the output control function 137 outputs a variety of information. For example, the output control function 137 performs control to display a variety of information in a display device. More particularly, the output control function 137 displays the user interface 71 in the display 120. The user interface 71 is used to allow the operator to confirm or adjust the imaging parameters that are set for capturing the imaging range related to the second organ in the second imaging method and that are estimated by the estimation function 136.

Moreover, the output control function 137 controls the communication interface 110 and sends the imaging parameters, which have been confirmed or adjusted by the operator, to the medical image diagnostic apparatus 200 that takes images of the subject according to the second imaging method (in the first embodiment, transesophageal echocardiography).

In the medical image diagnostic apparatus 200 that has received the imaging parameters from the image processing apparatus 100, the processing circuitry (not illustrated) sets the received imaging parameters as the imaging parameters for the subject according to the second imaging method, and takes images of the subject according to the second imaging method. In this case, the processing circuitry represents an example of a setting unit and an executing unit.

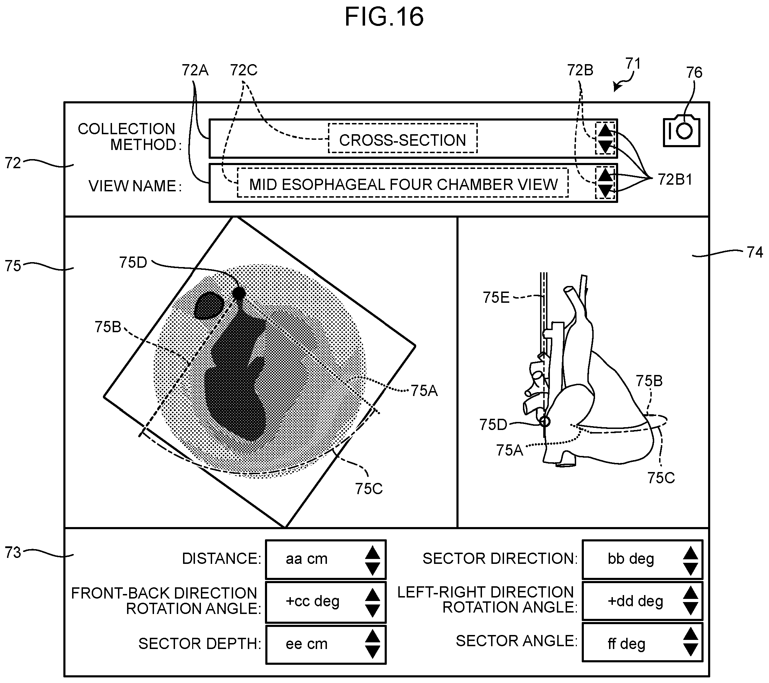

Regarding the output control function 137 and the user interface 71, the explanation is given below with reference to FIG. 16. FIG. 16 is a schematic diagram illustrating an example of the user interface 71 according to the first embodiment.

In the example illustrated in FIG. 16, the user interface 71 includes a display area 72 placed on the upper side; a display region 73 placed on the lower side; a display region 74 placed on the right side; and a display region 75 placed on the left side. The display regions 74 and 75 are placed in between the display regions 72 and 73.

In the user interface 71, in the display region 74 illustrated on the right side in FIG. 16, for example, a hear model is displayed that is generated based on the cardiac CT images obtained by the acquisition function 131. At a position corresponding to the heart model that is displayed in the display region 74, for example, the inner contour and the centerline of the esophagus, which is identified by the first feature identification function 132, can be displayed.

Moreover, at a position corresponding to the heart model that is displayed in the display region 74, for example, a virtual sector corresponding to the transducer position in the esophagus can be displayed based on the transducer position estimated by the transducer position estimation function 1362 and based on the sector depth (D2) and the section angle (A4) estimated by the parameter estimation function 1363. Furthermore, at a position corresponding to the heart model that is displayed in the display region 74, such information can be displayed which is obtained by expressing, on an image, various images parameters estimated by the parameter estimation function 1363.

Meanwhile, in the user interface 71, in the display area 72 illustrated on the upper side in FIG. 16, for example, display fields 72A regarding the collection method and the view name are displayed. For example, in a right portion 72B of the display fields 72A, a button 72B1 is displayed that allows the operator to select the collection method and the view name. Moreover, in a middle portion 72C of the display fields 72A, the collection method and the view name selected by the operator are displayed.

For example, when the operator operates the buttons 72B1, the view name list 72D and the cursor CU illustrated in FIG. 4 get displayed. That enables the operator to select the view name. Alternatively, when the operator operates the buttons 72B1, the view name list 72D1 illustrated in FIG. 5 can be displayed.

In the example illustrated in FIG. 16, in the display area 72, "cross-section" is displayed as the collection method selected by the operator, and "Mid Esophageal Four Chamber View" is displayed as the view name selected by the operator.

In the user interface 71, in the display region 74 illustrated on the left side in FIG. 16, for example, an MPR cross-section generated by the cross-section generation function 1361 is displayed.

In the user interface 71, in the display region 73 illustrated on the lower side in FIG. 16, for example, display fields are displayed regarding the imaging parameters that are estimated by the parameter estimation function 1363 and that correspond to the imaging range for the second organ (the heart of the subject) in the second imaging method (transesophageal echocardiography).

Thus, in the display region 73, display fields are displayed regarding the imaging parameters including the distance, the sector direction, the front-back direction rotation angle, the left-right direction rotation angle, the sector depth, and the section angle (D1 or D2, A1 to A4).

For example, in the display region 73, the distance, the sector direction, the front-back direction rotation angle, the left-right direction rotation angle, the sector depth, and the section angle are displayed as the imaging parameters. Moreover, in the right portion of the display fields, buttons are displayed for allowing the operator to adjust the distance, the sector direction, the front-back direction rotation angle, the left-right direction rotation angle, the sector depth, and the section angle. Furthermore, in the middle portion of the display fields are displayed the distance, the sector direction, the front-back direction rotation angle, the left-right direction rotation angle, the sector depth, and the section angle as selected by the operator.

Meanwhile, the imaging parameters such as the distance, the sector direction, the front-back direction rotation angle, the left-right direction rotation angle, the sector depth, and the section angle can be modified (adjusted) by the operator. For example, when the imaging parameters such as the distance, the sector direction, the front-back direction rotation angle, the left-right direction rotation angle, the sector depth, and the section angle are modified (adjusted) by the operator, the imaging parameters adjusted by the operator are displayed in the user interface 71.

In the example illustrated in FIG. 16, in the display region 73, the following imaging parameters that have been adjusted by the operator are displayed: distance "aacm"; front-back direction rotation angle "+bbdeg"; left-right direction rotation angle "+ccdeg"; sector direction "dddeg"; sector depth "eecm"; and sector angle "ffdeg".

Meanwhile, in the user interface 71, in the transesophageal echocardiography displayed in the display region 75, the two radii of the sectors can be displayed in a recognizable manner for the operator. For example, in the display region 75, the two radii of the sectors in the transesophageal echocardiography can be displayed as a red line 75A (a dotted line in FIG. 16) and a green line 75B (a dashed line in FIG. 16); and the arc joining those two radii can be displayed as a white line 75C (a dashed-dotted line in FIG. 16).

In the display region 74 too, using the heart model, the two radii of the sector can be displayed as the red line 75A (a dotted line in FIG. 16) and the green line 75B (a dashed line in FIG. 16); and the arc joining those two radii can be displayed as the white line 75C (a dashed-dotted line in FIG. 16).

In this way, in the display regions 74 and 75 of the user interface 71, as result of displaying the two radii of the sector in transesophageal echocardiography and displaying the arc joining the two radii, the operator becomes able to easily understand the sector direction with respect to the subject.

Moreover, in the user interface 71, the transducer position during transesophageal echocardiography as displayed in the display region 75, that is, the position of the light emitting element on the transducer can be displayed in a recognizable manner for the operator. For example, in the display region 75, the transducer position during transesophageal echocardiography can be displayed as a yellow point 75D.

Furthermore, in the display region 74, using the heart model, the centerline of the esophagus can be displayed as a yellow line 75E (a two-dot-dash line in FIG. 16).

In this way, in the display regions 74 and 75 in the user interface 71, as a result of displaying the transducer position during transesophageal echocardiography and displaying the centerline of the esophagus, the operator becomes able to more easily understand the transducer position.

Moreover, in the user interface 71, a save button 76 can also be displayed. When the operator presses the save button 76, the MPR cross-section estimated by the estimation function 136 can be stored in the memory 140 along with the imaging parameters including the distance, the sector direction, the front-back direction rotation angle, the left-right direction rotation angle, the sector depth, and the section angle.

For example, after selecting the most suitable imaging parameters using the user interface 71, the operator can press the save button 76 and store the most suitable imaging parameters in the memory 140.

Meanwhile, the content for display using the user interface 71 is not limited to the explanation given above. Thus, other imaging parameters or content confirmed or adjusted by the operator can also be displayed.

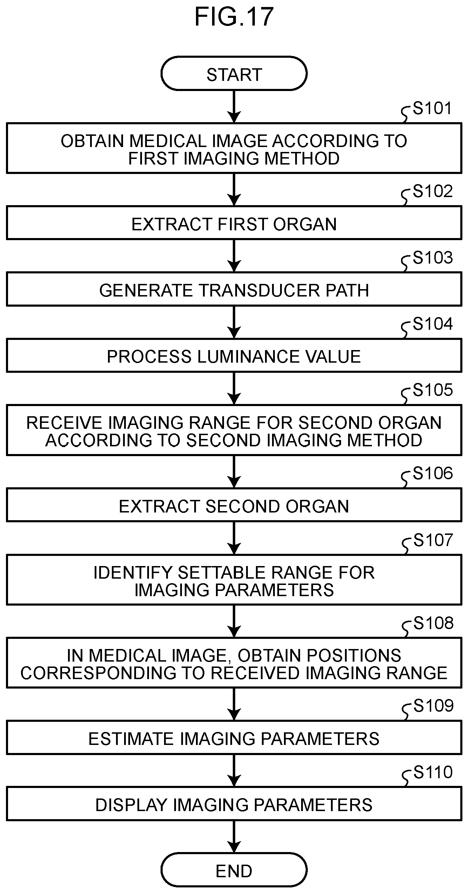

Explained below with reference to FIG. 17 are the operations performed by the image processing apparatus 100 according to the first embodiment. FIG. 17 is a flowchart for explaining an example of the operations performed by the image processing apparatus 100 according to the first embodiment.

Firstly, the acquisition function 131 obtains the first image data obtained according to the first imaging method (Step S101). For example, the acquisition function 131 obtains, as the first image data obtained according to the first imaging method, a cardiac CT image from an external apparatus such as an X-ray CT apparatus or a PACS server.

Then, the first feature identification function 132 extracts the first organ in the first image data (Step S102).

For example, the first feature identification function 132 identifies the inner contour and the centerline of the esophagus, which is captured in the cardiac CT image, as the features related to the first organ. Then, based on the identified inner contour and the identified centerline of the esophagus captured in the cardiac CT image, the first extraction function 1321 of the first feature identification function 132 extracts the region indicating the inner contour and the centerline of the esophagus captured in the cardiac CT image.

Subsequently, based on the first organ that has been segmented, the transducer path generation function 1322 of the first feature identification function 132 generates, in the first image data, a transducer path for the second imaging method (Step S103).

For example, based on the extracted inner contour and the extracted centerline of the esophagus, the transducer path generation function 1322 generates a transducer path for transesophageal echocardiography in the cardiac CT image.

Then, the image conversion function 138 processes the luminance value in the first image data in such a way that the luminance value in the first image data is converted into the luminance value according to the second imaging method (Step S104). For example, the image conversion function 138 converts the luminance value of a cardiac CT image (i.e., a CT image) into the luminance value of a medical image obtained during transesophageal echocardiography (i.e., an ultrasound image).

Subsequently, the reception function 133 receives the imaging range that is related to the second organ and that is for the second image data obtained according to the second imaging method (Step S105). For example, the reception function 133 receives, from the operator via the user interface 71, the selection input of the view name during transesophageal echocardiography.

Then, the second feature identification function 134 extracts the second organ in the first image data (Step S106).

For example, the second feature identification function 134 identifies, as the features related to the second organ, the region indicating the entire heart and the important structures therein as captured in the cardiac CT image.
Then, based on the identified region indicating the entire heart and the important structures therein as captured in the cardiac CT image, the second extraction function 1341 of the second feature identification function 134 extracts, the cardiac CT image, the region indicating the entire heart and the important structures therein.

Subsequently, the third feature identification function 135 identifies the settable range for imaging parameters in transesophageal echocardiography (Step S107). For example, based on at least either the constraints for transesophageal echocardiography, or the features of the esophagus representing the first organ that are extracted at Step S102, or the features of the heart representing the second organ that are extracted at Step S106; the third feature identification function 135 identifies the settable range for imaging parameters including D1 and D2 and A1 to A4.

Then, the cross-section generation function 1361 of the estimation function 136 obtains the positions in the medical image that correspond to the received imaging range for transesophageal echocardiography (Step S108). For example, the cross-section generation function 1361 of the estimation function 136 uses a learning model, which is trained using a known machine learning technology and which is capable of identifying three landmarks corresponding to the view name for transesophageal echocardiography, and identifies positions in the medical image that correspond to the imaging range for transesophageal echocardiography as received at Step S105.

Subsequently, based on the features related to the imaging range for the first organ and the features related to the imaging range for the second organ and based on the settable range for imaging parameters in the second imaging method, the parameter estimation function 1363 of the estimation function 136 estimates the imaging parameters in the second imaging method to be implemented for capturing the imaging range for second organ (Step S109).

For example, the transducer position estimation function 1362 of the estimation function 136 estimates, as the position of the transducer TD, the point of intersection between the esophagus, which is extracted at Step S102, and the cross-section in the cardiac CT image as identified from the position in the medical image identified at Step S108. Then, based on the estimated position of the transducer TD, the parameter estimation function 1363 estimates the distance D1 as the imaging parameter.

Moreover, the parameter estimation function 1363 estimates the imaging parameters including D2 and A1 to A4 based on the positional relationship between the estimated position of the transducer TD, the position of the esophagus in the cardiac CT image as extracted at Step S102, and the position of the heart in the cardiac CT image as extracted at Step S106, and based on the settable range for imaging parameters in the second imaging method as identified at Step S107.

The output control function 137 displays, in the display 120, the imaging parameters in the second imaging method that is implemented for capturing the imaging range related to the second organ (Step S110). That marks the end of the operations.

For example, in the display 120, the output control function 137 displays, as the initial values in the display region 73 of the user interface 71, the values of the distance D1, the sector direction A1, the front-back direction rotation angle A2, the left-right direction rotation angle A3, the sector depth D2, and the sector angle A4 at the time of performing transesophageal echocardiography with respect to the imaging range related to the heart as estimated at Step S109.

Moreover, for example, alongside displaying the imaging parameters, the output control function 137 displays, in the display 120 and as the initial value in the display region 75 of the user interface 71 illustrated in FIG. 16, the cross-section of the cardiac CT image as identified from the position in the medical image identified at Step S108.

As explained above, the image processing apparatus 100 according to the first embodiment obtains the first image data obtained according to the first imaging method; identifies the features related to the first organ captured in the first image data; identifies the features related to the imaging range for the second organ in the first image data that corresponds to the imaging range for the second organ in the second image data obtained according to the second imaging method; identifies the settable range for imaging parameters in the second imaging method; and, based on the features related to the first organ in the first image data, based on the features of the imaging parameters for the second organ, and based on the settable range for imaging parameters in the second imaging method, estimates the imaging parameters corresponding to the imaging range for the second organ in the second imaging method.

In this way, in the image processing apparatus 100 according to the first embodiment, based on the features related to the first organ (the esophagus of the subject) in the first image data (a cardiac CT image of the subject) and based on the features related to the imaging range for the second organ (the heart of the subject), it becomes possible to estimate the imaging parameters in the second imaging method. In that case, if the operator specifies the imaging range related to the second organ to be captured according to the second imaging method, the image processing apparatus 100 according to the first embodiment can automatically estimate the imaging parameters to be used in capturing the imaging range. As a result, the time and efforts required by the operator can be reduced, and the work efficiency can be enhanced. Hence, the operator can be provided with the preoperative planning in an efficient manner. Moreover, the image processing apparatus 100 according to the first embodiment can identify the settable range for imaging parameters and can estimate the imaging parameters by taking into account the settable range for imaging parameters. Herein, the settable range for imaging parameters implies the range of the parameters that can be actually captured in the second imaging method. Thus, the image processing apparatus 100 according to the first embodiment enables preventing estimation of such imaging parameters which are not actually capturable.

### Second embodiment

In the first embodiment explained earlier, the explanation is given about the case in which the image processing apparatus 100 performs the operations for estimating the imaging parameters. In a second embodiment, the explanation is given about the case in which the medical image diagnostic apparatus 200 performs the operations for estimating the imaging parameters.

The following explanation is mainly given about the differences with the first embodiment, and the detailed explanation about the already-explained points is not given again. Meanwhile, the embodiments descried below either can be implemented individually or can be combined in an appropriate manner.

Firstly, the explanation is given about a image processing system 1 according to the second embodiment.

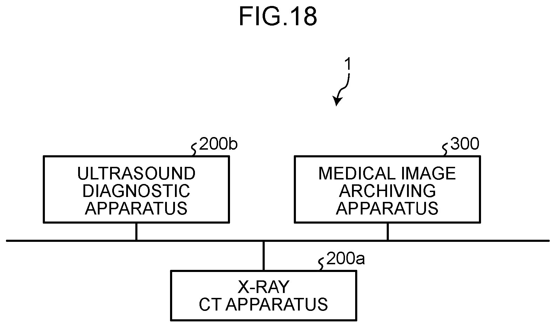

FIG. 18 is a block diagram illustrating an exemplary configuration of the image processing system 1 according to the second embodiment. As illustrated in FIG. 18, the image processing system 1 includes an X-ray CT apparatus 200a, an ultrasound diagnostic apparatus 200b, and the medical image archiving apparatus 300. The X-ray CT apparatus 200a, the ultrasound diagnostic apparatus 200b, and the medical image archiving apparatus 300 are connected to each other via a network.

The X-ray CT apparatus 200a and the ultrasound diagnostic apparatus 200b represent an example of an image diagnostic apparatus. Meanwhile, the image processing system 1 can include a plurality of X-ray CT apparatuses 200a and a plurality of ultrasound diagnostic apparatuses 200b.

Explained below with reference to FIG. 19 is a configuration of the X-ray CT apparatus 200a. FIG. 19 is a block diagram illustrating an exemplary configuration of the X-ray CT apparatus 200a according to the second embodiment. As illustrated in FIG. 19, the X-ray CT apparatus 200a includes a mount apparatus 10, a couch apparatus 30, and a console apparatus 40.

In the second embodiment, the longitudinal direction of the rotation axis of a rotatable frame 13 in the non-tilted state is defined as the Z-axis direction; the direction that is orthogonal to the Z-axis direction and that is oriented from the center of rotation toward a columnar support meant for supporting the rotatable frame 13 is defined as the X-axis; and the direction that is orthogonal to the Z-axis and the X-axis is defined as the Y-axis.

The mount apparatus 10 includes an imaging system 19 for taking medical images that are to be used in making a diagnosis. The imaging system 19 is configured with, for example, an X-ray tube 11, an X-ray detector 12, a wedge 16, and a collimator 17. Thus, the mount apparatus 10 includes the imaging system 19 that bombards X-rays onto a subject P and collects projection data from the detection data about the X-rays that have passed through the subject P.

The mount apparatus 10 has an opening portion formed to accommodate the subject P. A couchtop 33 on which the subject P is asked to lie down is housed inside the opening portion, with the side on which the couch apparatus 30 is disposed serving as the entry side.

The mount apparatus 10 includes the X-ray tube 11, the wedge 16, the collimator 17, the X-ray detector 12, an X-ray high-voltage generator 14, a data acquisition system (DAS) 18, the rotatable frame 13, a controller 15, and the couch apparatus 30.

The X-ray tube 11 is a vacuum tube that receives the application of a high voltage from the X-ray high-voltage generator 14 and that generates X-rays by bombarding thermal electrons from a cathode (filament) onto an anode (target). For example, the X-ray tube 11 is a rotating anode X-ray tube that generates X-rays by bombarding thermal electrons onto a rotating anode.

The wedge 16 is a filter for adjusting the X-ray dosage of the X-rays that are bombarded from the X-ray tube 11. More particularly, the wedge 16 is a filter that transmits and attenuates the X-rays, which are bombarded from the X-ray tube 11, in such a way that the X-rays bombarded from the X-ray tube 11 onto the subject P have a predetermined distribution.

For example, the wedge 16 is a wedge filter or a bow-tie filter, and is manufactured by processing aluminum to achieve a predetermined target angle and a predetermined thickness.

The collimator 17 is a lead plate meant for limiting the X-rays, which have passed through the wedge 16, within an X-ray bombardment range; and constitutes a slit due to a combination of a plurality of lead plates. The collimator 17 is sometimes also called an X-ray limiter.

The X-ray detector 12 detects the X-rays that were bombarded from the X-ray tube 11 and that have passed through the subject P; and outputs, to the DAS 18, an electrical signal corresponding to the X-ray dosage. The X-ray detector 12 includes, for example, a plurality of detection element arrays in each of which a plurality of X-ray detection elements is arranged in the channel direction along a single circular arc centered on the focal point of the X-ray tube 11. The channel direction implies the circumferential direction of the rotatable frame 13.

For example, the X-ray detector 12 has a structure in which a plurality of X-ray detection element arrays, in each of which a plurality of X-ray detection elements is arranged in the channel direction, is arranged along the slice direction (also called the body axis direction or the row direction).

Moreover, the X-ray detector 12 is, for example, an indirect-transform-type detector that includes a grid, a scintillator array, and an optical sensor array. The scintillator array includes a plurality of scintillators, each of which includes a scintillator crystal that outputs a light having the photon quantity according to the incident X-ray dosage. The grid is disposed on that face of the scintillator array which is on the X-ray incidence side, and includes an X-ray shield plate equipped to absorb the scattered X-rays.

The optical sensor array has the function of converting the light from the scintillator into electric signals according to the light intensity; and includes an optical sensor such as a photomultiplier tube (PMT). Meanwhile, alternatively, the X-ray detector 12 can be a direct-transform-type detector that includes a semiconductor element for converting the incident X-rays into electrical signals.

The X-ray high-voltage generator 14 includes electrical circuitry such as a transformer and a rectifier, and also includes a high-voltage generator having the function of generating a high voltage to be applied onto the X-ray tube 11 and an X-ray controller for controlling the output voltage according to the X-rays bombarded from the X-ray tube 11.
The high-voltage generator can be of the transformer type or the inverter type.

The X-ray high-voltage generator 14 either can be disposed in the rotatable frame 13, or can be disposed in the fixed frame (not illustrated) of the mount apparatus 10. The fixed frame is used in rotatably supporting the rotatable frame 13.

The DAS 18 includes an amplifier for performing amplification with respect to the electrical signals output from each X-ray detection element of the X-ray detector 12 and includes an A/D converter for converting the amplified electrical signals into digital signals; and generates detection data. The detection data generated by the DAS 18 is transferred to the console apparatus 40. The detection data is, for example, in the form of a sinogram.

A sinogram represents data in which, the projection data generated for each position of the X-ray tube 11 (hereinafter, also called the view angle) and for each X-ray detection element is illustrated in a corresponding manner to the view direction and the channel direction. The view direction corresponds to the view angle and implies the X-ray bombardment direction.

When one-time scanning is performed using only a single detection element array in the X-ray detector 12, it becomes possible to generate a single sinogram for a single instance of scanning. When helical scanning or volume scanning is performed using a plurality of detection element arrays in the X-ray detector 12, it becomes possible to generate a plurality of sinograms for a single instance of scanning.

The rotatable frame 13 is an annular frame that supports the X-ray tube 11 and the X-ray detector 12 opposite to each other and that rotates the X-ray tube 11 and the X-ray detector 12 under the control of the controller 15. Meanwhile, in addition to supporting the X-ray tube 11 and the X-ray detector 12, the rotatable frame 13 also supports the X-ray high-voltage generator 14 and the DAS 18.

The rotatable frame 13 is rotatably supported by the non-rotatable portion of the mount apparatus 10 (for example, supported by a fixed frame (not illustrated in FIG. 19)). Herein, the rotation mechanism includes, for example, a motor that generates a rotary driving force and a bearing that transmits the rotary driving force to the rotatable frame 13 and causes the rotatable frame 13 to rotate. The motor is disposed in, for example, the non-rotatable portion. The bearing is physically connected to the rotatable frame 13 and the motor, and causes the rotatable frame 13 to rotate according to the torque of the motor.

In the rotatable frame 13 as well as in the non-rotatable portion, communication circuitry is disposed that is either of the contactless type or of the contact type. As a result, the units that are supported by the rotatable frame 13 can communicate with the non-rotatable portion or with external apparatuses of the mount apparatus 10.

For example, when optical communication is implemented as the contactless communication method, the detection data generated by the DAS 18 is sent using optical communication from a transmitter, which is disposed in the rotatable frame 13 and which includes a light emitting diode (LED), to a receiver, which is disposed in the non-rotatable portion of the mount apparatus 10 and which includes a photodiode. Moreover, the detection data is transferred from the non-rotatable portion to the console apparatus 40 using the transmitter.

Meanwhile, as the communication method, it is possible to implement contactless data transfer using the capacitive coupling method or the radio wave method, or it is possible to implement a contact-type data transmission method using a slip ring and an electrode brush.

The controller 15 includes processing circuitry including a central processing unit (CPU), and includes a driving mechanism including a motor and an actuator. The controller 15 receives an input signal from an input interface 43 (explained later) that is attached to the console apparatus 40 or the mount apparatus 10, and accordingly controls the operations of the mount apparatus 10 and the couch apparatus 30.

For example, the controller 15 receives an input signal and performs control to rotate the rotatable frame 13, or performs control to tilt the mount apparatus 10, or performs control to operate the couch apparatus 30 and the couchtop 33. Regarding the control for tilting the mount apparatus 10, according to inclination angle (tilt angle) information input via the input interface attached to the mount apparatus 10, the controller 15 rotates the rotatable frame 13 around the axis parallel to the X-axis direction.

Meanwhile, the controller 15 either can be disposed in the mount apparatus 10, or can be disposed in the console apparatus 40.

The couch apparatus 30 is an apparatus on which the subject P, who is the target for scanning, is made to lie down and is moved. The couch apparatus 30 includes a base 31, a couch driving apparatus 32, the couchtop 33, and a supporting frame 34. The base 31 is a housing that supports the supporting frame 34 to be movable in the vertical direction. The couch driving apparatus 32 is a motor or an actuator that moves the couchtop 33, on which the subject P is lying down, in the long-axis direction of the couchtop 33 (in FIG. 19, the Z-axis direction).

The couchtop 33 is a plate placed on the upper surface of the supporting frame 34, and the subject P is asked to lie down on the couchtop 33. Meanwhile, in addition to moving the couchtop 33, the couch driving apparatus 32 can also move the supporting frame 34 in the long-axis direction of the couchtop 33.

The couch driving apparatus 32 moves the base 31 in the vertical direction according to a control signal from the controller 15. Moreover, the couch driving apparatus 32 moves the couchtop 33 in the long-axis direction (the Z-axis direction) according to a control signal from the controller 15.

The console apparatus 40 receives an operation performed by the operator with respect to the X-ray CT apparatus 200a, and reconstructs the X-ray CT image data from the X-ray detection data collected by the mount apparatus 10. The console apparatus 40 includes a memory 41, a display 42, the input interface 43, and processing circuitry 45.

The memory 41 is implemented, for example, using a semiconductor memory device such as a random access memory (RAM) or a flash memory, or using a hard disk, or using an optical disc. The memory 41 is used to store, for example, projection data and reconstructed image data. Moreover, the memory 41 is used to store the imaging protocol.

In the imaging protocol, a procedure for controlling the imaging system 19 and taking images of the subject P is specified. For example, the imaging protocol represents a group of parameters such as the body part to be subjected to imaging, the imaging conditions, the imaging range, the reconstruction conditions, the operation of the mount apparatus 10 (the imaging system 19), and the operation of the couch apparatus 30.

The memory 41 is used to store dedicated computer programs meant for implementing a system control function 451 (explained later), a preprocessing function 452 (explained later), a reconstruction processing function 453 (explained later), an image processing function 454 (explained later), the acquisition function 131, the first feature identification function 132, the reception function 133, the second feature identification function 134, the third feature identification function 135, the estimation function 136, the output control function 137, and the image conversion function 138.

The display 42 is a monitor referred to by the operator and is used to display a variety of information. For example, the display 42 is used to output medical images (CT images) generated by the processing circuitry 45, and to output a graphical user interface (GUI) meant for receiving various operations from the operator. Examples of the display 42 include a liquid crystal display or a cathode ray tube (CRT) display.

Returning to the explanation with reference to FIG. 19, the input interface 43 receives various input operations from the operator, converts the input operations into electrical signals, and outputs the electrical signals to the processing circuitry 45. For example, from the operator, the input interface 43 receives collection conditions to be implemented at the time of collecting the projection data, or receives reconstruction conditions to be implemented at the time of reconstructing the CT image data, or receives image processing conditions to be implemented at the time of generating a postprocessing image from a CT image.

The input interface 43 is implemented using, for example, a mouse, a keyboard, a trackball, switches, buttons, or a joystick. Meanwhile, the input interface 43 can alternatively be disposed in the mount apparatus 10. Still alternatively, the input interface 43 can be configured using a tablet terminal capable of performing wireless communication with the console apparatus 40.

The processing circuitry 45 controls the operations of the entire X-ray CT apparatus 200a. For example, the processing circuitry 45 includes the system control function 451, the preprocessing function 452, the reconstruction processing function 453, the image processing function 454, the acquisition function 131, the first feature identification function 132, the reception function 133, the second feature identification function 134, the third feature identification function 135, the estimation function 136, the output control function 137, and the image conversion function 138.

In the second embodiment, the processing functions implemented by the constituent elements, namely, the system control function 451, the preprocessing function 452, the reconstruction processing function 453, the image processing function 454, the acquisition function 131, the first feature identification function 132, the reception function 133, the second feature identification function 134, the third feature identification function 135, the estimation function 136, the output control function 137, and the image conversion function 138 are stored in the memory 41 in the form of computer programs executable in a computer. The processing circuitry 45 is a processor that reads the computer programs from the memory 41 and executes them so as to implement the function corresponding to each computer program.

In other words, after having read the computer programs, the processing circuitry 45 becomes equipped with the functions as illustrated in the processing circuitry 45 in FIG. 19.

With reference to FIG. 19, it is explained that a single unit of processing circuitry 45 is equipped with the processing functions implemented by the system control function 451, the preprocessing function 452, the reconstruction processing function 453, the image processing function 454, the acquisition function 131, the first feature identification function 132, the reception function 133, the second feature identification function 134, the third feature identification function 135, the estimation function 136, the output control function 137, and the image conversion function 138. Alternatively, the processing circuitry 45 can be configured by combining a plurality of independent processors, and each processor can be made to execute a computer program and implement the corresponding function.

In other words, either the configuration can be such that each function is configured as a computer program and a single unit of processing circuitry executes all computer programs, or the configuration can be such that specific functions are installed in a program execution circuitry that is dedicated and independent in nature.

Based on an input operation received from the operator via the input interface 43, the system control function 451 controls various functions of the processing circuitry 45. For example, via the input interface 43, the system control function 451 receives input of user information (for example, the user ID) required for the login and receives input of subject information. Moreover, for example, the system control function 451 receives input of the imaging protocol via the input interface 43.

The preprocessing function 452 generates data as a result of performing preprocessing such as logarithmic conversion or offset correction, inter-channel sensitivity correction, and beam hardening correction with respect to the detection data output from the DAS 18. Herein, the data (detection data) before performing preprocessing and the data having been subjected to preprocessing is sometimes collectively referred to as projection data.

The reconstruction processing function 453 performs reconstruction processing with respect to the projection data, which is generated by the preprocessing function 452, using the filtered back projection method or the successive approximation reconstruction method according to the reconstruction conditions; and generates a plurality of sets of slice image data (CT image data).

The image processing function 454 performs postprocessing with respect to a plurality of sets of slice image data generated by the reconstruction processing function 453, and generates postprocessing image data. The data before performing preprocessing (i.e., a plurality of sets of slice image data) and the data having been subjected to preprocessing is sometimes collectively referred to as X-ray CT image data.

The postprocessing defined herein represents the concept of performing the processing with respect to a plurality of sets of slice image data generated by the reconstruction processing function 453. For example, the postprocessing includes noise removal, multi-planar reconstruction (MPR) display of a plurality of sets of slice image data, and rendering of volume data.

For example, based on an input operation received from the operator via the input interface 43, the image processing function 454 implements a known method and converts the X-ray CT image data, which is generated by the reconstruction processing function 453, into cross-sectional image data or an arbitrary cross-section or into three-dimensional image data. Meanwhile, alternatively, the three-dimensional image data can be directly generated by the reconstruction processing function 453.

The acquisition function 131, the first feature identification function 132, the reception function 133, the second feature identification function 134, the third feature identification function 135, the estimation function 136, the output control function 137, and the image conversion function 138 are identical to the first embodiment. Hence, their detailed explanation is given again.

Meanwhile, in the second embodiment, the acquisition function 131 can obtain the X-ray CT image data, which is stored in the memory 41, as the first image data.

Moreover, in the second embodiment, the output control function 137 controls the communication interface (not illustrated) included in the console apparatus 40, and sends the imaging parameters, which have been confirmed or adjusted by the operator, to the ultrasound diagnostic apparatus 200b.

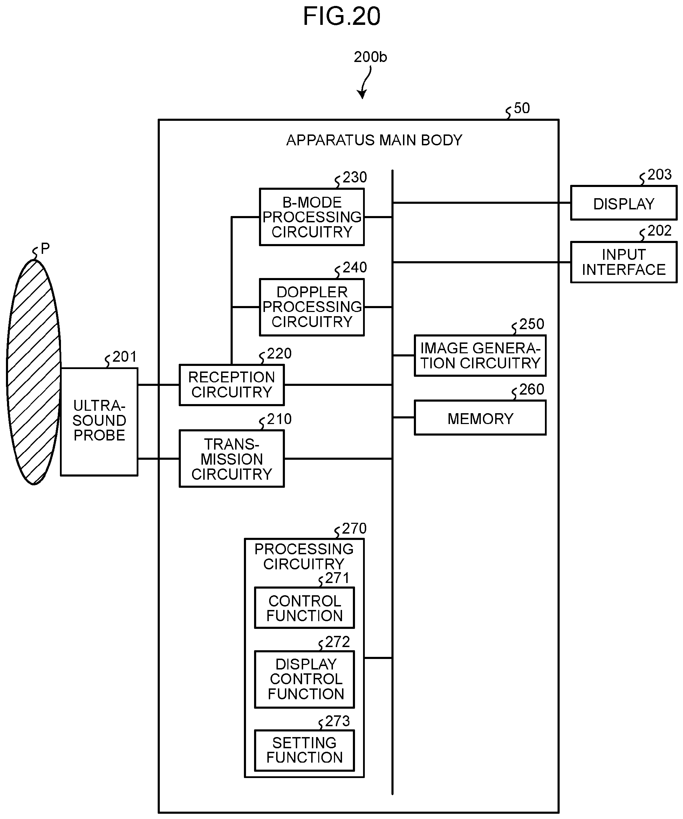

Explained below with reference to FIG. 20 is a configuration of the ultrasound diagnostic apparatus 200b. FIG. 20 is a block diagram illustrating an exemplary configuration of the ultrasound diagnostic apparatus 200b according to the second embodiment. As illustrated in FIG. 20, the ultrasound diagnostic apparatus 200b according to the second embodiment includes an apparatus main body 50, an ultrasound probe 201, an input interface 202, and a display 203.

Moreover, as illustrated in FIG. 20, the ultrasound probe 201, the ultrasound probe 201, the input interface 202, and the display 203 are connected to the apparatus main body 50.

The ultrasound probe 201 includes a plurality of oscillators (piezoelectric vibrators). The ultrasound probe 201 is touched to the body surface of the subject P, and performs ultrasonic transmission (ultrasound scanning). The oscillators generate ultrasonic waves based on a driving signal supplied from a transmission circuitry 210 (explained later). The generated ultrasonic waves get reflected from the acoustic impedance mismatch interface in the subject P, and are received by the oscillators as reflected wave signals (received echo) including the components scattered due to the scattering bodies in the tissue. Then, the ultrasound probe 201 sends the reflected wave signals, which are received by the oscillators, to a reception circuitry 220.

Herein, the ultrasound probe 201 that is connected to the apparatus main body 50 can be changed as appropriate. For example, depending on the target body part for inspection in the subject P, the operator such as a doctor or a technician selects one of a plurality of ultrasound probes 201 connectible to the apparatus main body 50, and connects it to the apparatus main body 50. In the second embodiment, a TEE probe is used as the ultrasound probe 201.

The ultrasound probe 201 connected to the apparatus main body 50 either can be a two-dimensional ultrasound probe (a 2D array probe) including a plurality of oscillators arranged in a matrix (lattice), or can be a one-dimensional ultrasound probe (1D array probe) including a plurality of oscillators arranged one-dimensionally in a predetermined direction. Alternatively, a plurality of ultrasound probes 201 can be connected to the apparatus main body 50.

The input interface 202 receives a variety of input operations from the operator, converts each received input operation into electrical signals, and outputs the electrical signals to a processing circuitry 270. For example, after the ultrasound diagnostic apparatus 200b is turned on, the input interface 202 receives an operation performed by the operator for logging in.

Moreover, for example, regarding the patient to be examined, the input interface 202 receives an input operation for patient information (patient ID, gender of the patient, age of the patient, past examination results, and doctor's findings). The input interface 202 is implemented using, for example, a trackball used for various settings, or switch buttons, or a mouse, or a joystick, or a touchpad enabling the input by touching on the operation screen, or a touchscreen configured by integrating a display screen and a touchpad, or a contactless input circuit in which an optical sensor is used, or a sound input circuit.

However, the input interface 202 is not limited to include a physical operation component such as a mouse or a keyboard. For example, an electrical signal processing circuitry that receives electrical signals corresponding to an input operation from an external input apparatus, which is provided as a separate apparatus from the apparatus main body 50, and outputs the electrical signals to the processing circuitry is also considered as an example of the input interface 202.

The display 203 displays a graphical user interface (GUI) meant for enabling the operator of the ultrasound diagnostic apparatus 200b to input various setting requests from, and displays ultrasound image data that is generated in the apparatus main body 50.

For example, the display 203 is configured using, for example, a liquid crystal monitor or a cathode ray tube (CRT) display. The display 203 can be of the desktop type, or can be configured with a tablet terminal capable of performing wireless communication with the apparatus main body 50.

The apparatus main body 50 generates ultrasound image data based on reflected wave signals received by the ultrasound probe 201. For example, as illustrated in FIG. 20, the apparatus main body 50 includes the transmission circuitry 210, the reception circuitry 220, a B-mode processing circuitry 230, a doppler processing circuitry 240, an image generation circuitry 250, a memory 260, and a processing circuitry 270.

The transmission circuitry 210, the reception circuitry 220, the B-mode processing circuitry 230, the doppler processing circuitry 240, the image generation circuitry 250, the memory 260, and the processing circuitry 270 are communicably connected to each other.

The transmission circuitry 210 includes a pulse circuitry that repeatedly generates the pulse rate, which is meant for forming ultrasound transmission waves, at a predetermined pulse repetition frequency (PRF), and outputs the generated pulse rate to the ultrasound probe 201.

Moreover, the pulse circuitry applies the driving signal (driving pulse) to the ultrasound probe 201 at a timing based on the pulse rate. Moreover, under the control performed by the processing circuitry 270, the transmission circuitry 210 sends, to the ultrasound probe 201, a delay amount with respect to the ultrasonic waves transmitted from the ultrasound probe 201.

The reception circuitry 220 includes an A/D converter and a receiving beam former. For example, after the reflected wave signals are received from the ultrasound probe 201, the A/D converter converts the reflected wave signals into digital data. Then, the receiving beam former performs delay-and-sum beamforming with respect to the digital data coming from each channel, and generates reflected wave data. Subsequently, the receiving beam former sends the reflected wave data to the B-mode processing circuitry 230 and the doppler processing circuitry 240.

The B-mode processing circuitry 230 receives the reflected wave data output from the reception circuitry 220; performs logarithmic amplification and envelop detection with respect to the reflected wave data; and generates data (B-mode data) in which the signal intensity is expressed as luminance.

The doppler processing circuitry receives the reflected wave data output from the reception circuitry 220; performs frequency analysis of speed information from the reflected wave data; extracts the blood flow and the tissue attributed to the doppler effect and extracts the ultrasound contrast agent; ang generates data (doppler data) in which mobility information such as average velocity, dispersion, and power is extracted for a large number of points.

The image generation circuitry 250 generates ultrasound image data from the data generated by the B-mode processing circuitry 230 and the doppler processing circuitry 240. For example, from the B-mode data generated by the B-mode processing circuitry 230, the image generation circuitry 250 generates B-mode image data in which the intensity of the reflected wave is expressed as luminance.

Moreover, for example, from the doppler data generated by the doppler processing circuitry 240, the image generation circuitry 250 generates doppler image data representing the mobility information. The doppler image data represents velocity image data, dispersion image data, power image data, or image data obtained combining those sets of image data.

Meanwhile, the image generation circuitry 250 can also perform a variety of other processing with respect to the ultrasound image data. For example, the image generation circuitry 250 performs coordinate transformation according to the manner of scanning of the ultrasonic waves by the ultrasound probe 201, and generates ultrasound image data for display.

As an example, the image generation circuitry 250 converts the scanning line signal sequence of ultrasound scanning into the scanning line signal sequence of a video format represented by the television (i.e., performs scan conversion); and generates ultrasound image data for display.

Moreover, for example, using a plurality of image frames obtained after scan conversion, the image generation circuitry 250 performs image processing for regenerating an average luminance image (i.e., performs smoothing) and performs image processing in which a derivative filter is used in the images (i.e., performs edge enhancement). Furthermore, for example, the image generation circuitry 250 merges the character information of a variety of parameters, the scales, and the bodily marks in the ultrasound image data.

The memory 260 is implemented using, for example, a semiconductor memory device such as a random access memory (RAM) or a flash memory; or a hard disk; or an optical disk. For example, the memory 260 stores therein the data generated by the B-mode processing circuitry 230 and the doppler processing circuitry 240 (i.e., the B-mode data and the doppler data), and stores therein the image data generated by the image generation circuitry 250 (the B-mode image data and the doppler image data).

Moreover, for example, the memory 260 stores therein a variety of data such as the patient information, the diagnostics protocol, and various bodily marks. Furthermore, for example, the memory 260 stores therein a computer program enabling the circuitry included in the ultrasound diagnostic apparatus 200b to implement its functions. Meanwhile, the memory 260 can be implemented using a server group (cloud) that is connected to the ultrasound diagnostic apparatus 200b via a network.

The processing circuitry 270 controls the overall operations of the ultrasound diagnostic apparatus 200b. For example, the processing circuitry 270 implements a control function 271, a display control function 272, and a setting function 273.

The control function 271 controls various functions of the processing circuitry 270 based on the input operations received from the operator via the input interface 202. For example, based on various setting requests received from the operator, the control function 271 controls the operation of the transmission circuitry 210, the reception circuitry 220, the B-mode processing circuitry 230, the doppler processing circuitry 240 and the image generation circuitry 250; and collects ultrasound image data.

The display control function 272 displays the ultrasound image data for display, which is generated by the image generation circuitry 250, in the display 203.

The setting function 273 sets the imaging parameters for transesophageal echocardiography. The setting function 273 represents an example of a setting unit. For example, the setting function 273 controls the communication interface (not illustrated) included in the apparatus main body 50, and receives the imaging parameters sent from the X-ray CT apparatus 200a. Then, the setting function 273 sets the received imaging parameters as the imaging parameters for performing transesophageal echocardiography with respect to the subject P.

Then, the control function 271 controls the constituent elements of the ultrasound diagnostic apparatus 200b and, according to the imaging parameters that are set, performs transesophageal echocardiography with respect to the subject P. In that case, the control function 271 represents an example of an executing unit.

In the ultrasound diagnostic apparatus 200b illustrated in FIG. 20, the processing functions are stored as computer-executable programs in the memory 260. Each circuitry illustrated in FIG. 20 is a processor that reads a computer program from the memory 260 and executes it so as to implement the function corresponding to that computer program. In other words, after having read a computer program, a processing circuitry becomes equipped with the function corresponding to that computer program.

Meanwhile, with reference to FIG. 20, the case is illustrated in which the control function 271, the display control function 272, and the setting function 273 are implemented by the single processing circuitry 270. However, the second embodiment is not limited to that case. Alternatively, for example, the processing circuitry 270 can be configured by combining a plurality of independent processors, and each processor can execute a computer program and implement the corresponding processing function. Still alternatively, the processing functions of the processing circuitry 270 can be dispersed among a plurality of circuitry or can be integrated into a single circuitry.

Till now, the explanation was given about the case in which the single memory 260 stores therein a computer program corresponding the function of each circuitry illustrated in FIG. 20. However, the second embodiment is not limited to that case. Alternatively, it is possible to have a plurality of memories 260 disposed in a dispersed manner.

In that case, the circuitry illustrated in FIG. 20 reads the computer programs from the individual memories 260 and implement the corresponding functions. Still alternatively, instead of storing the computer programs in the memory 260, the computer programs can be directly embedded into the circuitry of the processor. In that case, the processor reads the computer programs embedded into the circuitry, and implements the corresponding functions.

Meanwhile, each circuitry illustrated in FIG. 20 can implement the corresponding function using the processor of an external apparatus connected via a network. For example, along with reading the computer programs corresponding to the functions illustrated in FIG. 20 from the memory 260 and executing them, the processing circuitry 270 uses an external workstation, which is connected to the ultrasonic diagnostic apparatus 200b via a network, or a server group (cloud) as the computation resource and implements the functions.

According to the second embodiment, the operations related to the estimation of the parameters can be performed without requiring a separate image processing apparatus. For that reason, a system for performing the operations related to the estimation of the parameters can be built with a simple configuration.

Meanwhile, the embodiments described above can be modified by changing some part of the configuration or some of the functions of the image processing apparatus 100. The following explanation, modification examples of the first embodiment are described as other embodiments. The following explanation is mainly given about the differences with the first embodiment, and the detailed explanation about the already-explained points is not given again. Meanwhile, the modification examples descried below either can be implemented individually or can be combined in an appropriate manner.

### First modification example

In the first embodiment, the explanation is given about the case in which the third feature identification function 135 identifies the maximum length of the curve of the centerline of the esophagus as the settable range for the distance D1. However, the method by which the third feature identification function 135 identifies the settable range for the distance D1 is not limited to that method.

For example, the third feature identification function 135 can calculate the distance from the mouth of the subject to the esophageal entrance and the distance from esophageal exit to the connecting stomach wall on the side of the heart; and can identify the length obtained by adding those distances to the length of the esophagus as the settable range for the distance D1 representing an imaging parameter.

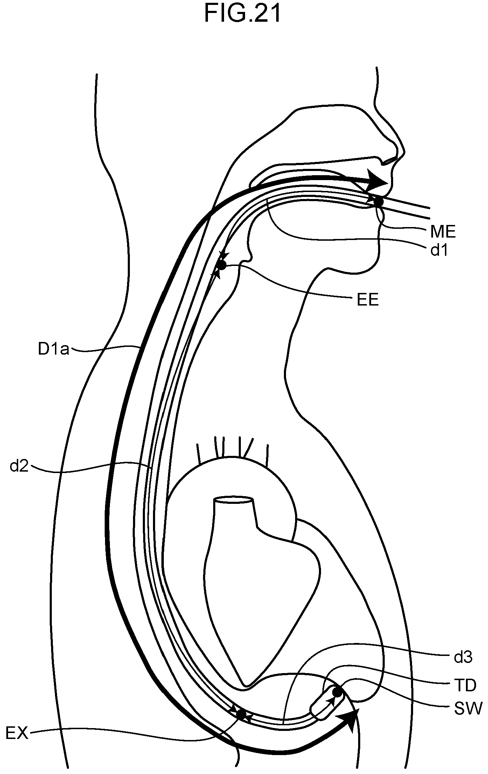

FIG. 21 is a diagram for explaining an example of an identification operation performed for identifying the settable range for the distance D1, which represents an imaging parameter, according to a first modification example. With reference to FIG. 21, when d1 represents the calculated distance from a mount ME of the subject to an esophageal entrance EE is calculated and when d3 represents the calculated distance from an esophageal exit EX to a connected stomach wall SW on the side of the heart is calculated, D1a represents the length obtained by adding the distances d1 and d3 to a length d2 of the esophagus (i.e., D1a represents d1+d2+d3). According to the first modification example, the third feature identification function 135 identifies the distance D1a as the settable range for the distance D1 representing an imaging parameter.

Alternatively, for example, the third feature identification function 135 can calculate the curvature at each position of the centerline of the esophagus; and, as the settable range for the distance D1 representing an imaging parameter, can identify the positions at which the curvature is within a prespecified settable curvature range.

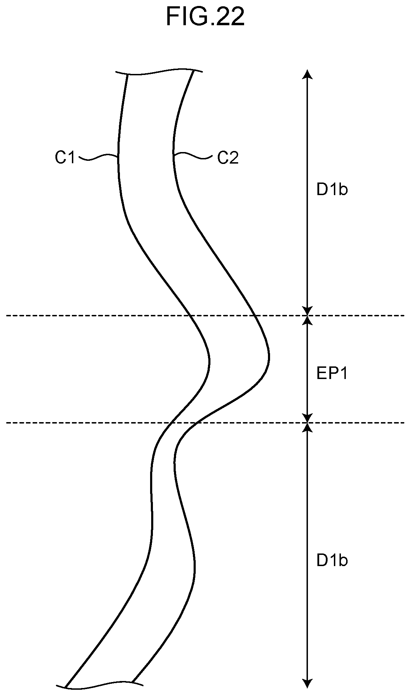

FIG. 22 is a diagram illustrating an example of the identification operation performed for identifying the settable range for the distance D1, which represents an imaging parameter, according to the first modification example. In an identical manner to FIG. 11, the curved lines C1 and C2 illustrated in FIG. 22 represent the inner contour of the esophagus in a particular cross-section. Moreover, in FIG. 22, an excluded portion EP1 represents the region in which the curvature of the centerline of the esophagus exceeds the prespecified settable curvature range.

According to the first modification example, the third feature identification function 135 excludes such distances from the mouth of the subject which fall within the excluded portion EP1 and, as the settable range for the distance D1 representing an imaging parameter, identifies the range of such distances from the mouth of the subject which fall within D1b.

Alternatively, for example, the third feature identification function 135 can calculate the diameter at each position of the esophagus and, as the settable range for the distance D1 representing an imaging parameter, can identify the positions at which the diameter is within a prespecified settable diameter range.

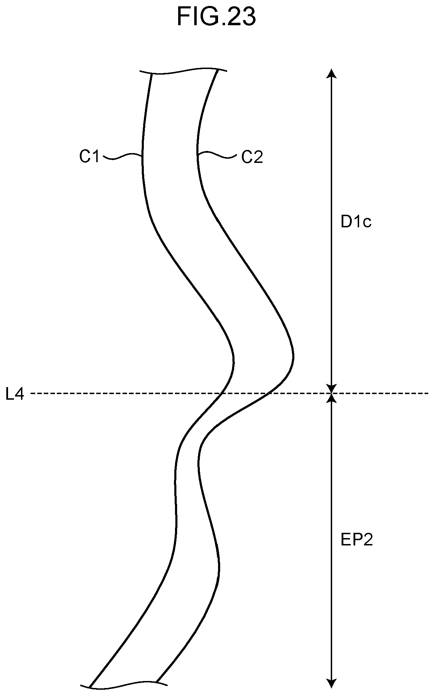

FIG. 23 is a diagram illustrating an example of the identification operation performed for identifying the settable range for the distance D1, which represents an imaging parameter, according to the first modification example. In an identical manner to FIG. 11, the curved lines C1 and C2 illustrated in FIG. 23 represent the inner contour of the esophagus in a particular cross-section. Moreover, a dotted line L4 represents the position at which the diameter is the longest within the settable diameter range. In FIG. 23, an excluded portion EP2 represents the region below the position at which the diameter of the centerline of the esophagus is the longest within the prespecified settable diameter range (i.e., below the dotted line L4) (thus, the excluded portion EP2 represents the region present on the side of the stomach with reference to the concerned position). That is, in the example illustrated in FIG. 23, regarding a position at which the diameter of the centerline of the esophagus is greater than the prespecified settable diameter range, if that position is below the dotted line L4 (on the side of the stomach), it is treated as part of the excluded portion EP2. Hence, in the example illustrated in FIG. 23, the region below the dotted line L4 (on the side of the stomach) is excluded from the settable range for the distance D1 representing an imaging parameter.

According to the first modification example, the third feature identification function 135 excludes such distances from the mouth of the subject which fall within the excluded portion EP2 and, as the settable range for the distance D1 representing an imaging parameter, identifies the range of such distances from the mouth of the subject which fall within D1c.

### Second modification example

In the first embodiment described earlier, the explanation is given about the case in which the reception function 133 receives the imaging range for transesophageal echocardiography via the view name list 72D displayed in the user interface 71. However, the method by which the reception function 133 receives the imaging range for transesophageal echocardiography is not limited to that method.

In the second modification example, the operator selects in advance a past test image of the same subject (for example, an ultrasound image obtained by performing transesophageal echocardiography) or selects in advance an image corresponding to the desired imaging range from among the test images of some other subjects; and inputs the selected image to the image processing apparatus 100. The reception function 133 receives, as the imaging range desired by the operator, the imaging range corresponding to the imaging range captured in the image input by the operator.

Moreover, at the time of generating an MPR cross-section, based on the similarity between the image input by the operator and the images capturing various imaging ranges in the cardiac CT images of the subject, the cross-section generation function 1361 identifies the imaging range in the CT images that corresponds to the imaging range desired by the operator.

For example, the cross-section generation function 1361 implements a known method and calculates the image features in the input image and in the images capturing various imaging ranges in the cardiac CT images of the subject, and calculates the similarity based on the magnitude of the difference in each image feature.

In the second modification example, for example, the actual images obtained by performing transesophageal echocardiography in the medical facility to which the operator is affiliated can be treated as the target images for input at the time of receiving the imaging range for transesophageal echocardiography. For that reason, according to the second modification example, it becomes possible to obtain the imaging range for the second image data, which is obtained according to the second imaging method, that is close to what the operator has in mind.

### Third modification example

In the first embodiment described earlier, the explanation is given about the case in which the parameter estimation function 1363 limits the search range based on the settable range for imaging parameters, and identifies the positions corresponding to the imaging range within only that search limit. In a third modification example, the explanation is given about a case in which the estimated imaging parameters are compared with the identified settable range for the imaging parameters, and it is determined whether the estimated imaging parameters are included in the settable range.

In the third modification example, at Step S109 in the flowchart illustrated in FIG. 17, after the imaging parameters are estimated, the parameter estimation function 1363 compares the estimated imaging parameters with the settable range for imaging parameters as identified by the third feature identification function 135, and determines whether the estimated imaging parameters are included in the settable range for imaging parameters.

If it is determined that the estimated imaging parameters are included in the settable range for imaging parameters, the parameter estimation function 1363 treats the estimated imaging parameters as the estimation result, and ends the operation at Step S109.

However, if it is determined that the estimated imaging parameters are not included in the settable range for imaging parameters, the system control returns to Step S108 in the flowchart illustrated in FIG. 17. In that case, at Step S108, the estimation function 136 (the cross-section generation function 1361) according to the third modification example performs control to identify such positions which are different than the positions identified as the positions of the cardiac CT images corresponding to the imaging range for transesophageal echocardiography received in the past.

More particularly, at Step S108, the estimation function 136 according to the third modification example stores, in the memory 140, the information related to the imaging range that is once identified before the system control proceeds to Step S109. Then, the estimation function 136 ensures that the imaging range recorded as once-identified imaging range is not identified again.

Meanwhile, in the case of re-identifying the imaging range at Step S108, the estimation function 136 can set a margin in advance and can ensure that re-identification is not performed for the imaging range obtained by adding the margin to the once-identified imaging range.

Meanwhile, it is a known fact the reliability of the information estimated using a known machine learning technology can be probabilistically calculated using the likelihood. Hence, at Step S108, the estimation function 136 can identify a plurality of candidates for the imaging range based on the likelihood. In that case, at Step S109, the estimation function 136 can compare the candidates for the imaging range in descending order of likelihood with the settable range for imaging parameters.

### Fourth modification example

According to the first embodiment or the third modification example, at Step S108 in the flowchart illustrated in FIG. 17, theoretically, there are times when no corresponding parameter is present. That is, there are times when, in the settable range for imaging parameters as identified at Step S107, the imaging parameters that enable imaging of such a range in the cardiac CT images which corresponds to the imaging range desired by the operation and received at Step S105 are not present (or cannot be identified theoretically).

In that case, the estimation function 136 can collaborate with the output control function 137 to display, in the display 120, a popup window for issuing a warning that "the corresponding imaging parameters are nonexistent".

Meanwhile, in the third modification example, it is possible to set a limit in advance on the number of iterations of Steps S108 and S109. In that case, the estimation function 136 can collaborate with the output control function 137 to display a warning as soon as the iteration limit is reached.

Meanwhile, the estimation function 136 can classify a plurality of imaging parameters into the imaging parameters present within the settable range for imaging parameters and the imaging parameters not present within the settable range for imaging parameters. In that case, the estimation function 136 can collaborate with the output control function 137 to display, at the time of issuing a warning to the operator, the estimated parameters along with the settable range for each parameter.

Alternatively, the estimation function 136 can collaborate with the output control function 137 to display, at the time of issuing a warning, only the parameters not present within the settable range for imaging parameters. Still alternatively, the estimation function 136 can collaborate with the output control function 137 to display, in a highlighted manner, the parameters not present within the settable range for imaging parameters.

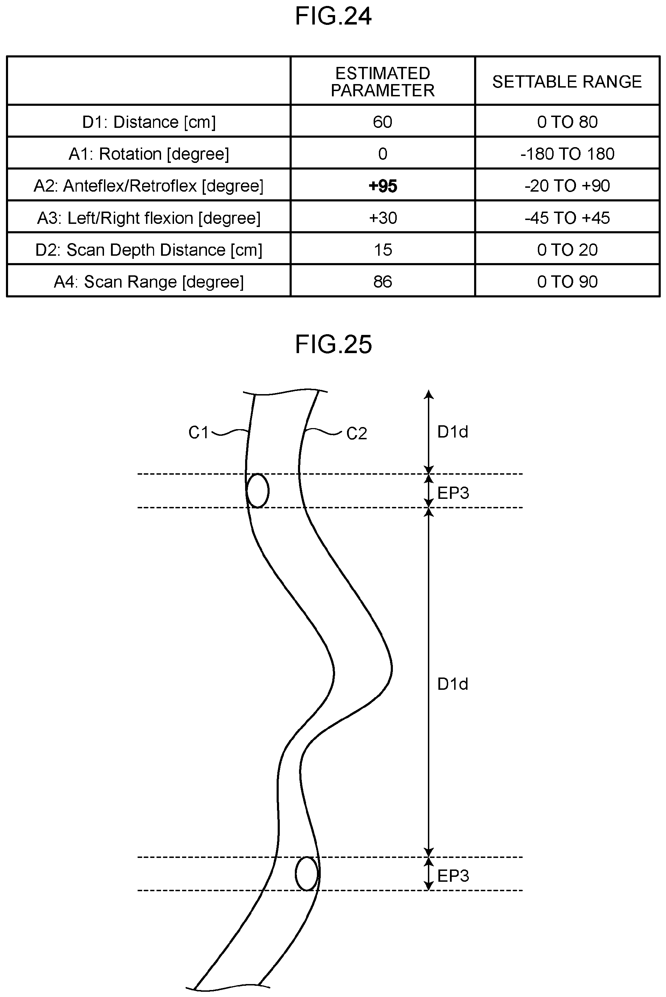

FIG. 24 is a diagram illustrating an example of the display operation for displaying the imaging parameters according to the fourth modification example. In the example illustrated in FIG. 24, the parameter A2 that is not present within the settable range for imaging parameters is displayed in a highlighted manner. Moreover, in the example illustrated in FIG. 24, each imaging parameter is displayed along with the corresponding setting range.

In this way, the operator is notified about the parameters not present within the settable range for imaging parameters along with the settable ranges for those parameters. As a result, the operator becomes able to easily adjust the imaging parameters to be within the range for transesophageal echocardiography.

### Fifth modification example

At Step S109 in the flowchart illustrated in FIG. 17, when a plurality of parameters satisfies the conditions, the estimation function 136 can collaborate with the output control function 137 to display the parameters, which satisfy the conditions, side by side.

Meanwhile, the estimation function 136 can collaborate with the output control function 137 to display only a single imaging parameter selected based on a predetermined condition. Examples of the predetermined condition include "D1 is the smallest" and "sum of A2 and A3 is the smallest".

The predetermined conditions mentioned above are only exemplary. Thus, as long as a condition can be identified based on at least either the estimated imaging parameters and the settable range for the imaging parameters, any condition can be treated as the predetermined condition.

Meanwhile, as explained in the third modification example, at Step S108, when a plurality of candidates for the imaging range is estimated, the estimation function can collaborate with the output control function 137 to perform simultaneous display of the imaging parameters corresponding to the imaging range. Moreover, the estimation function can collaborate with the output control function 137 to perform simultaneous display of the cross-sections in the cardiac CT images corresponding to the imaging range.

### Sixth modification example

In the first embodiment described earlier, the explanation is given about the case in which, at Step S107 illustrated in FIG. 17, the settable range is identified based on the anatomical structure of the first organ. In addition, it is also possible to perform an operation for identifying abnormalities inside or outside of the structure of the first organ. In that case, the third feature identification function 135 can identify the settable range based on the abnormalities (for example, while avoiding the abnormalities). Herein, an abnormality in the first organ represents an example of a region of interest of the first organ. In that case, the third feature identification function 135 represents an example of a "fifth feature identifying unit".

An abnormality can be detected using a known feature detection technology represented by CADe or by asking the operator to manually input the position of the abnormality.

FIG. 25 is a diagram illustrating an example of the identification operation for identifying the settable range for the distance D1, which represents an imaging parameter, according to a sixth modification example. In an identical manner to FIG. 11, the curved lines C1 and C2 illustrated in FIG. 25 represent the inner contour of the esophagus in a particular cross-section. Moreover, in FIG. 25, an excluded portion EP3 indicates an identified abnormality.

According to the sixth modification example, the third feature identification function 135 excludes such distances from the mouth of the subject which fall within the excluded portion EP3 and, as the settable range for the distance D1 representing an imaging parameter, identifies the range of such distances from the mouth of the subject which fall within D1d.

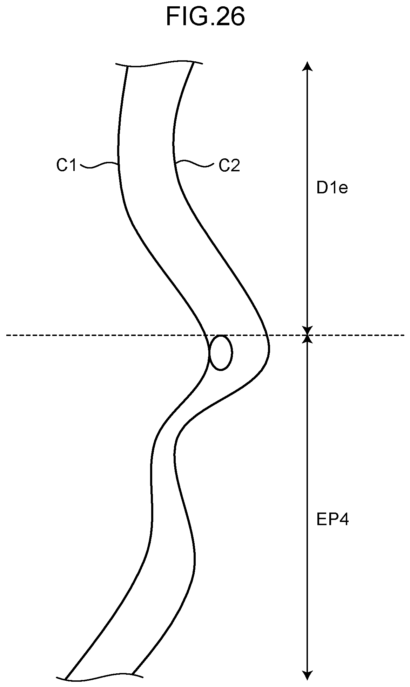

FIG. 26 is a diagram illustrating another example of the identification operation for identifying the settable range for the distance D1, which represents an imaging parameter, according to the sixth modification example. In an identical manner to FIG. 11, the curved lines C1 and C2 illustrated in FIG. 26 represent the inner contour of the esophagus in a particular cross-section. Moreover, in FIG. 26, an excluded portion EP4 represents that the region of the esophagus which is present below an identified abnormality. As such, the third feature identification function 135 may set the excluded portion EP4 by including some region surrounding the identified abnormality. The third feature identification function 135 may then exclude such distances from the mouth of the subject which fall within the excluded portion EP4, and identify the range of such distances from the mouth of the subject which fall within D1e, as the settable range for the distance D1 representing an imaging parameter.

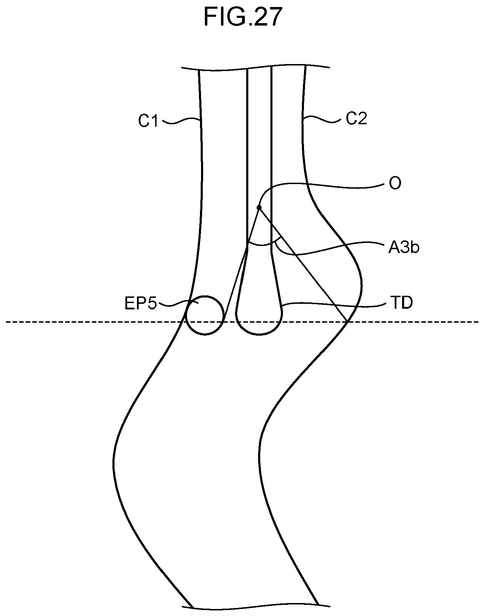

FIG. 27 is a diagram for explaining an example of the identification operation for identifying the settable range for the left-right direction rotation angle A3, which represents an imaging parameter, according to the sixth modification example. In an identical manner to FIG. 11, the curved lines C1 and C2 illustrated in FIG. 27 represent the inner contour of the esophagus in a particular cross-section. In FIG. 27, an excluded portion EP5 represents an identified abnormality.

According to the sixth modification example, the third feature identification function 135 excludes such left-right direction rotation angles which fall within the excluded portion EP5 and, as the settable range for the left-right direction rotation angle A3 representing an imaging parameter, identifies the range of such left-right direction rotation angles which fall within A3b.

According to the sixth modification example, for example, estimation of the imaging parameters can be performed only after removing such imaging parameters due to which the transducer TD is likely to get positioned at a position at which it not practically impossible (or difficult) to place the transducer TD because of the presence of an abnormality in the esophagus of the subject.

### Seventh modification example

Based on the imaging range identified at Step S108 or the imaging range estimated from the imaging parameters estimated at Step S109 in the flowchart illustrated in FIG. 17, the cardiac CT image can be clipped (or cropped) only around that imaging range. In a seventh modification example, the processing circuitry 130 receives input of the cropped image and performs the operations from Step S102 onward.

In the seventh modification example, in the case of extracting the first organ or the second organ, the analysis target becomes localized. With respect to a localized region, more accurate analysis can be performed. Hence, according to the seventh modification example, in the case of extracting the first organ or the second organ, it becomes possible to perform more accurate analysis.

### Eighth modification example

In the first embodiment described earlier, the image processing apparatus and the image processing method are explained with reference to the example of transesophageal echocardiography. However, the first embodiment is not limited to that case. Alternatively, for example, the image processing apparatus and the image processing method according to the first embodiment can be used in an intra cardiac echo catheter (ICE).

### Ninth embodiment

In the first embodiment described earlier, the explanation is given about the case in which the CT imaging method represents the first imaging method, transesophageal echocardiography represents the second imaging method, a CT image represents the first image data, and an ultrasound image represents the second image data.

However, the first embodiment is not limited to that case. Alternatively, for example, the first imaging method can be magnetic resonance imaging (MRI), and the second imaging method can be angiography.

In that case, an MRI image represents the first image data, and an angiogram represents the second image data. Still alternatively, the first imaging method can be CT imaging, and the second imaging method can be angiography.

### 10-th modification example

In the first embodiment described earlier, the explanation is given about the case in which the esophagus represents the first organ, and the heart represents the second organ. However, the first embodiment is not limited to that case. Alternatively, the first organ and the second organ can be some other organs of the subject.

Herein, when the first image data based on the first imaging method has correspondence relationship with the second image data based on the second imaging method and when the imaging range and the imaging parameters in the second imaging method are variable in the imaging process according to the second imaging method, the image processing apparatus and the image processing method according to the first embodiment can be applied for arbitrary medical images.

### 11-th modification example

In the first embodiment described earlier, the explanation is given about the case in which the method for extracting an organ from the image data can be the Otsu's method based on CT values, the region growing method, the snake method, the graph cut method, the mean shift clustering, or a known machine learning technology. However, the method for extracting an organ from the image data is not limited to those examples. Alternatively, for example, as long as the position of the target organ can be identified in an image, any arbitrary method can be implemented.

### 12-th modification example

In the first embodiment described earlier, the explanation is given about the case in which D1 and D2 as well as A1 to A4 represent the imaging parameters for transesophageal echocardiography. However, the imaging parameters for transesophageal echocardiography are not limited to those parameters. Alternatively, for example, an angle A5 in the beam direction (hereinafter, also simply referred to as A5) can also be treated as an imaging parameter.

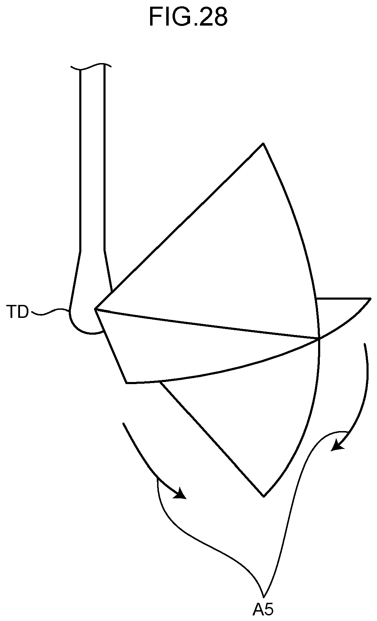

FIG. 28 is a diagram for explaining an example of the imaging parameter according to the 12-th modification example. As illustrated in FIG. 28, the angle A5 represents the angle of the scan range in the beam direction that is perpendicular to the transceiver surface of the transducer TD.

The term "processor" mentioned above implies, for example, a central processing unit (CPU), a graphics processing unit (GPU), or a circuitry such as an application specific integrated circuit (ASIC) or a programmable logic device (for example, a simple programmable logic device (SPLD), a complex programmable logic device (CPLD), or a field programmable gate array (FPGA)).

The constituent elements of the apparatuses illustrated in the drawings are merely conceptual, and need not be physically configured as illustrated. The constituent elements, as a whole or in part, can be separated or integrated either functionally or physically based on various types of loads or use conditions. Moreover, the process functions implemented in the apparatuses are entirely or partially implemented by a CPU or by computer programs that are analyzed and executed by a CPU, or are implemented as hardware by wired logic.

Meanwhile, the image processing method described in the embodiments can be implemented by executing a computer program written in advance in a computer such as a personal computer or a workstation. The computer program can be distributed over a network such as the Internet. Alternatively, the computer program can be stored in a non-transitory computer-readable recording medium such as a hard disk, a flexible disk (FD), a compact disk read only memory (CD-ROM), a magneto-optical (MO) disk, or a digital versatile disk (DVD). Thus, a computer can read the computer program from the recording medium and execute it.

According to at least one of the embodiments described above, it becomes possible to support the acquisition of appropriate medical images.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the inventions as defined by the appended claims. The accompanying claims and are intended to cover such forms or modifications as would fall within the scope of the inventions.

## Claims

1. An image processing apparatus (100) comprising:
a first acquisition unit (131) configured to obtain first image data captured according to a first imaging method;
a first feature identification unit (132) configured to identify a first feature related to a first organ captured in the first image data;
a second feature identification unit (134) configured to identify, corresponding to imaging range for a second organ that is different than the first organ and that is captured in second image data obtained according to a second imaging method different than the first imaging method, a feature related to imaging range for the second organ in the first image data;
a third feature identification unit (135) configured to identify a settable range for an imaging parameter in the second imaging method; and
an estimation unit (136) configured to estimate based on the first feature related to the first organ in the first image data, the second feature related to imaging range for the second organ, and the settable range for an imaging parameter in the second imaging method, an imaging parameter for the second organ in the second imaging method.

2. The image processing apparatus (100), further comprising:
a reception unit (133) configured to receive imaging range for the second organ.

3. The image processing apparatus (100) according to claim 1, wherein
the third feature identification unit (135) is further configured to identify the settable range for the imaging parameter based on the first feature related to the first organ in the first image data.

4. The image processing apparatus (100) according to claim 1, wherein
the third feature identification unit (135) is further configured to identify the settable range for the imaging parameter based on relative position of the first organ and the second organ in the first image data.

5. The image processing apparatus (100) according to claim 1, further comprising:
a fourth feature identification unit (134) configured to identify the second feature related to the second organ in the first image data, wherein
the third feature identification unit (135) is further configured to identify the settable range for the imaging parameter, based on the second feature related to the second organ in the first image data.

6. The image processing apparatus (100) according to claim 1, further comprising:
a fifth feature identification unit (135) configured to identify position of a region of interest in the first organ in the first image data, wherein
the third feature identification unit (135) is further configured to identify the settable range for the imaging parameter, based on position of the region of interest in the first organ in the first image data.

7. The image processing apparatus (100) according to claim 1, further comprising:
a second acquisition unit (135) configured to obtain constraint information related to a constraint on an apparatus used for the second imaging method, wherein
the third feature identification unit (135) is further configured to identify the settable range for the imaging parameter, based on the constraint information.

8. The image processing apparatus (100) according to claim 1, wherein
the estimation unit (136) is further configured to identify a cross-section corresponding to imaging range for the second organ in the first image data, and accordingly estimate an imaging parameter corresponding to the second feature related to imaging range for the second organ.

9. The image processing apparatus (100) according to claim 1, wherein
the estimation unit (136) is further configured to estimate a provisional imaging parameter, based on the first feature related to the first organ in the first image data and based on the second feature related to imaging range for the second organ,
when the provisional imaging parameter is within the settable range for the imaging parameter, the estimation unit (136) estimates the provisional parameter to be the imaging parameter, and
when the provisional imaging parameter is outside the settable range for the imaging parameter, the estimation unit (136) excludes the provisional parameter and again performs operation of estimating the provisional imaging parameter.

10. The image processing apparatus (100) according to claim 9, wherein
the second feature identification unit (135) is further configured to newly identify the second feature related to imaging range for the second organ in such a way that, when the provisional imaging parameter is outside the settable range for the imaging parameter, the second feature related to imaging range for the second organ becomes different than a previously-identified second feature related to imaging range for the second organ, and
the estimation unit (136) is further configured to estimate the provisional imaging parameter based on the newly-identified second feature related to imaging range for the second organ.

11. The image processing apparatus (100) according to any one of claims 1 to 10, wherein
the first imaging method indicates imaging performed using an X-ray CT apparatus, and the first image data indicates CT image data obtained by an X-ray CT apparatus, and
the second imaging method indicates imaging according to transesophageal echocardiography, and the second image data indicates ultrasound image data obtained during transesophageal echocardiography.

12. The image processing apparatus (100) according to any one of claims 1 to 11, wherein
the first organ indicates esophagus, and the second organ indicates heart.

13. The image processing apparatus (100) according to any one of claims 1 to 12, further comprising:
an output unit (136) configured to output the estimated imaging parameter to an image diagnostic apparatus that performs imaging according to the second imaging method.

14. An image processing system comprising:
the image processing apparatus (100) according to claim 13; and
the image diagnostic apparatus according to claim 13, wherein
the image diagnostic apparatus includes
a setting unit configured to set the imaging parameter output from the image processing apparatus as an imaging parameter for performing imaging according to the second imaging method, and
an execution unit configured to perform imaging according to the second imaging method using the set imaging parameter.

15. An image processing method implemented in an image processing apparatus, comprising:
obtaining first image data according to a first imaging method,
identifying a first feature related to a first organ captured in the first image data,
identifying, corresponding to imaging range for a second organ that is different than the first organ and that is captured in second image data obtained according to a second imaging method different than the first imaging method, a second feature related to imaging range for the second organ in the first image data,
identifying a settable range for an imaging parameter in the second imaging method, and
estimating based on the feature related to the first organ in the first image data, the feature related to imaging range for the second organ, and the settable range for an imaging parameter in the second imaging method, an imaging parameter for the second organ in the second imaging method.
